# EUROPEAN PATENT SPECIFICATION

(11) **EP 2 007 901 B1**
(45) Date of publication and mention of the grant of the patent: **09.08.2017**
(21) Application number: 07720681.1
(22) Date of filing: 04.04.2007
(51) Int. Cl.: C12Q 1/68

(54) **RAPID GENOTYPING ANALYSIS AND THE DEVICE THEREOF**
SCHNELLE GENOTYPISIERUNGSANALYSE UND VORRICHTUNG DAFÜR
ANALYSE RAPIDE DE GENOTYPAGE ET DISPOSITIF ASSOCIE

(30) Priority: 04.04.2006 US 398433
(43) Date of publication of application: 31.12.2008
(73) Proprietor: Guangzhou Baigao Medical Laboratory Co., Ltd., Haizhu District, Guangzhou City (CN)
(72) Inventor: TAM, Joseph, Wing, On, Hong Kong (CN)
(74) Representative: inCompass IP Europe Limited
(86) International application number: PCT/CN2007/001108
(87) International publication number: WO 2007/115491

(56) References cited:
- EP-A1- 0 540 997
- WO-A1-03/100095
- WO-A2-2004/023092
- US-A- 5 741 647
- US-A- 6 020 187
- US-A1- 2004 023 248
- US-A1- 2004 185 452
- US-A1- 2004 209 253
- US-A1- 2005 079 493

## Description

### BACKGROUND OF THE PRESENT INVENTION

### Field of Invention

The present invention relates to a method of making rapid, definitive identification of human leukocytic antigens (HLA) by DNA analysis and the device thereof. The present invention also relates to a method of making rapid, definitive identification of a human or any organism by DNA analysis and the device thereof.

### Description of Related Arts

Accurate HLA typing is essential for matching donor and recipient in organ or marrow transplantation (Thomas, 1983) to prevent the development of acute graft-versus-host disease (GVHD). This is generally accomplished by standard serological typing (Mach et al., 1990). Recent studies have demonstrated that DNA genotyping can provide more accurate and definitive result (Kaneshige et. al., (1993); Chow and Tonai, 2003; Mach et al., 2004). Results of HLA-DQ, DR and DP genotyping provided data for accurate matching which is necessary in selecting potential organ donors (Tam, 1998; 2004; 2005; 2006).

HLA genotyping using sequence-specific primers polymerase chain reaction (SSP-PCR) amplifications has been previously reported. However, due to highly polymorphic nature of HLA-DQ, DR and DP loci, the number of SSP required will be in the hundreds, and, therefore, exceed the limit of multiplex-PCR for efficient PCR amplification. A kit that is available on the market today includes an amplification process of carrying out a 96 PCR separate reactions, then followed by analysis on gel electrophoresis size separation. This is not only time consuming and costly but also prone to error due to the complexity of the reaction setup and sizing uncertainty of gel electrophoresis.

Thus, DNA sequencing is still considered the method of choice for accurate genotyping of the HLA cluster. Unfortunately, because of the existence of highly homologous sequence of pseudogene(s) that may be co-amplified during the polymerase chain reaction (PCR) amplification process, accurate genotyping by DNA sequencing alone may prove more difficult and costly.

The present invention discloses a method of rapidly analyzing HLA loci of DP, DR and DQ beta sequences by Allelic-Specific-Oligonucleotide (ASO) probes using an improved flow-through format. Such flow-through format, which was described in U.S. Patent Nos. 5,471,547 and 6,020,187, employs a fast annealing process that uses a very inexpensive device for accurate mutation detection, genotyping and fingerprinting analysis.

DNA fingerprinting by restriction fragment length polymorphism (RFLP) was first introduced in 1985 (Gill P, et al.) for human identification, and was subsequently applied to identification of other organisms. In practical applications, DNA fingerprinting has been widely accepted as the best forensic tool for identification of suspects in criminal cases, for paternity disputes and for establishing or verifying the identity of a person. The time consuming RFLP method has been gradually replaced by high throughput automated processes. Using PCR amplification for analyzing the number of short tandem repeat (STR), first discovered in 1991 (Edwards et al., 1991), from 10, 16, 18 or more loci in the human genome, single cell identification is now possible. However, both STR and/or variable number of tandem repeats (VNTR) are relatively expensive because these methods require the use of sophisticated equipment, and labor intensive and time consuming process like Southern blotting hybridization. Sporadic mutations (Chakraborty and Stivers, 1996) may reduce the accuracy and the power for definitive identification. Furthermore, .STR data suggested that the frequency of mutation, particularly in cancer patients, is not uncommon. Hence, new alternative method is needed.

US2004/209253 discloses a flow-through hybridization method for a nucleic acid assay for determination of the identity of a target nucleic acid. The target DNA molecules require amplification before the target molecule detection can take place. US5741647 discloses a lateral flow hybridization device for detecting target analytes of nucleic acid molecules, but one which requires an additional layer to effectively redirect the flow of the sample to achieve a more sensitive measurement. US2004/023248 discloses signal enhancement of a plurality of labeled interaction parameters. Antibiotin antibody-DNA conjugate for detection of target nucliec acid molecules is introduced, but not in a flow-through hybridization process.

Single nucleotide polymorphism (SNP) genotyping provides greater discriminating power by selecting an appropriate number of SNPs at unlink loci, and the mutation frequency in each locus (site) is lower than VNTR or STR systems for forensic or individual personal identification. The present invention provides a method of making rapid, definitive biometric - identification of an individual, such as a human being, animal, plant or any organism, using SNP genotyping.

### SUMMARY OF THE PRESENT INVENTION

The present invention describes Allelic-Specific-Oligonucleotide Reversed-Dot-Blotting (ASO-RDB) direct flow-through hybridization is a better alternative for the detection of specific target HLA DNA sequences. The data obtained refer to the specific segments of HLA loci of DP, DR and DQ beta that are able to provide accurate determination of the genotypes. The present invention provides a cost effective procedure for HLA identification by using common primer pairs to perform a simple multiplex PCR followed by hybridization with the required numbers of ASO-probes in a Low-Density Array format. The amplified HLA fragments (including the pseudogenes) can be analyzed in a single membrane embedded with the ASO-probes for definitive HLA classification. Hence, this is a far superior method than other DNA or serological methods currently available. This invention provides a HLA typing technique which is faster and simpler, does not require expensive equipment, and is therefore less costly to manufacture and operate than direct DNA sequencing and multiplex PCR gel electrophoresis procedures.

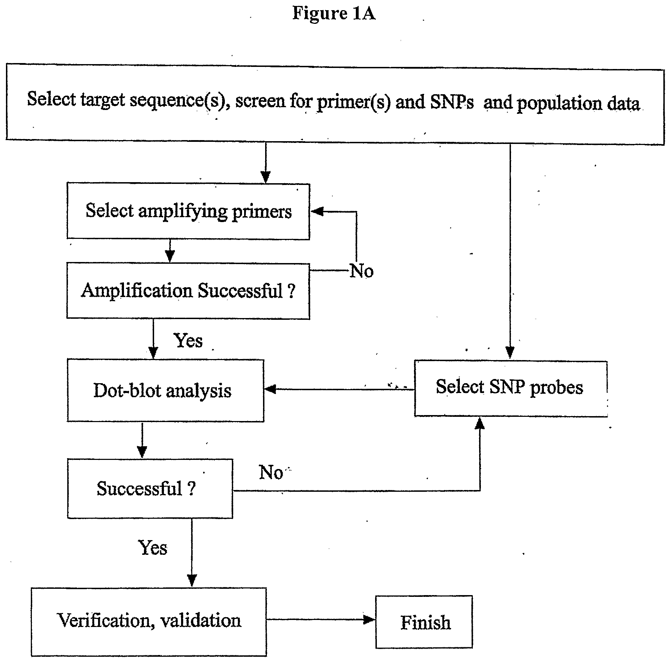

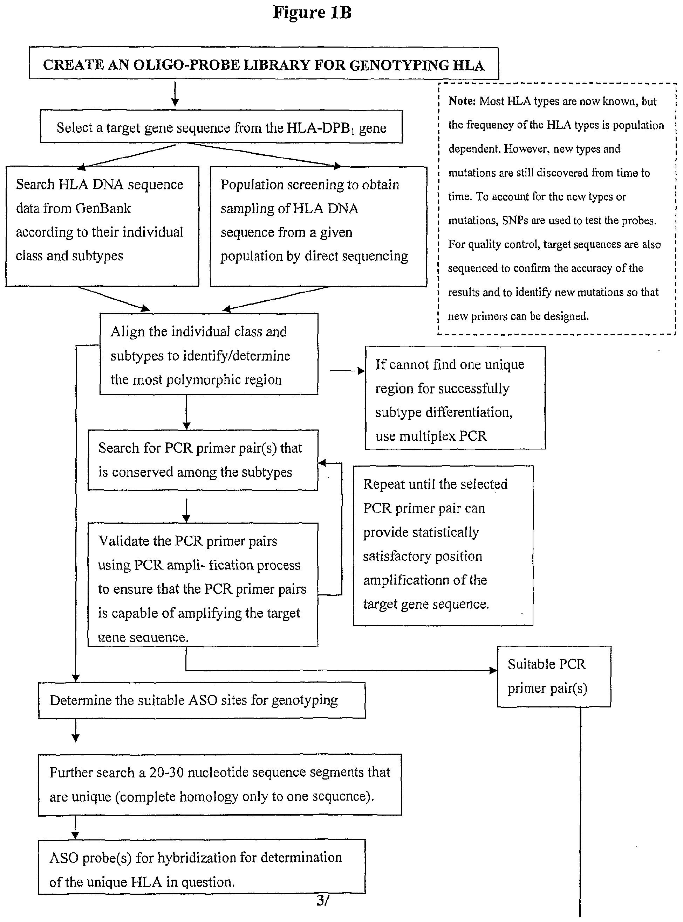

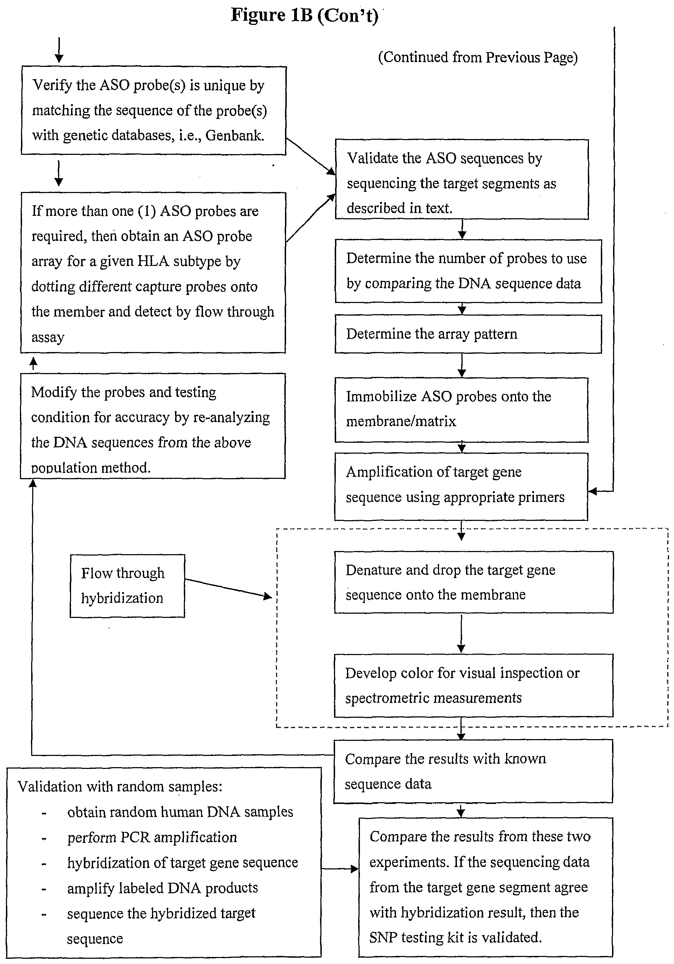

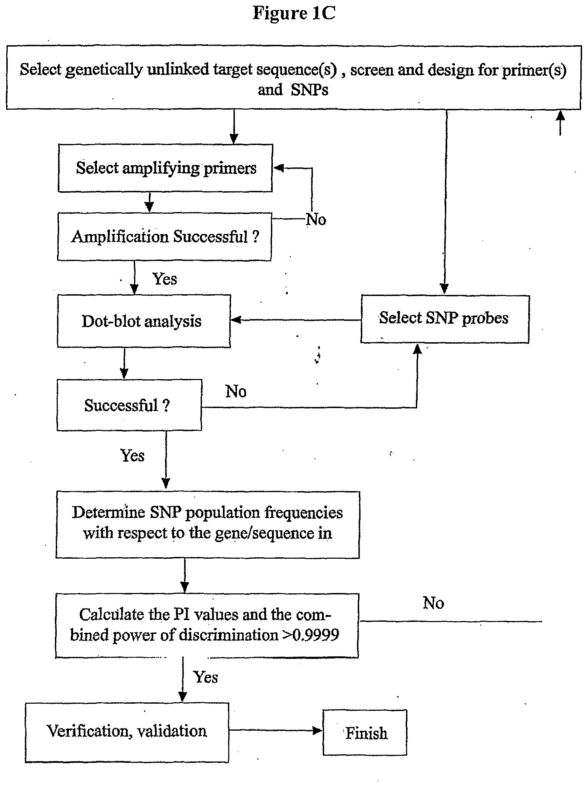

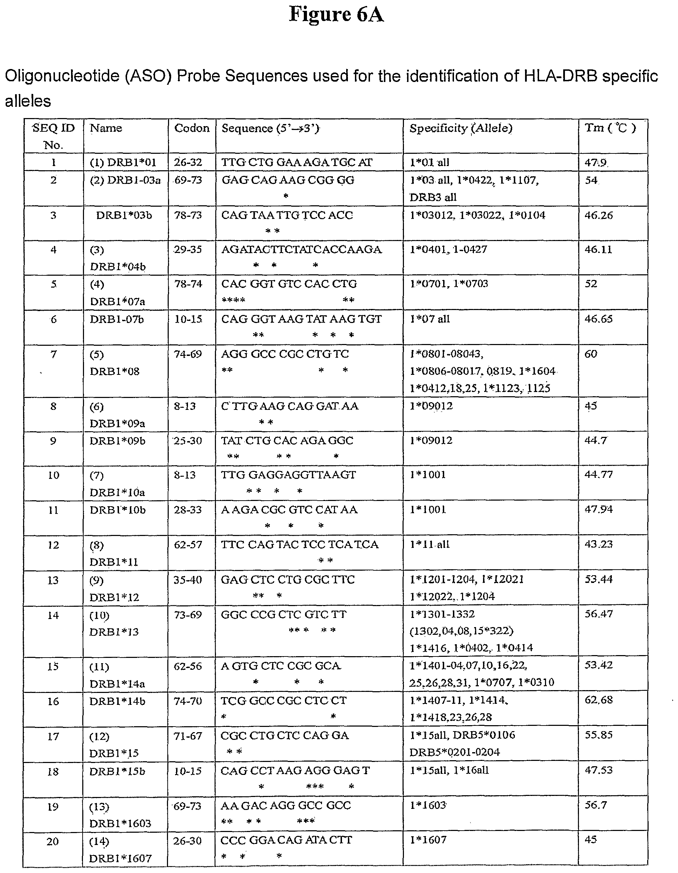

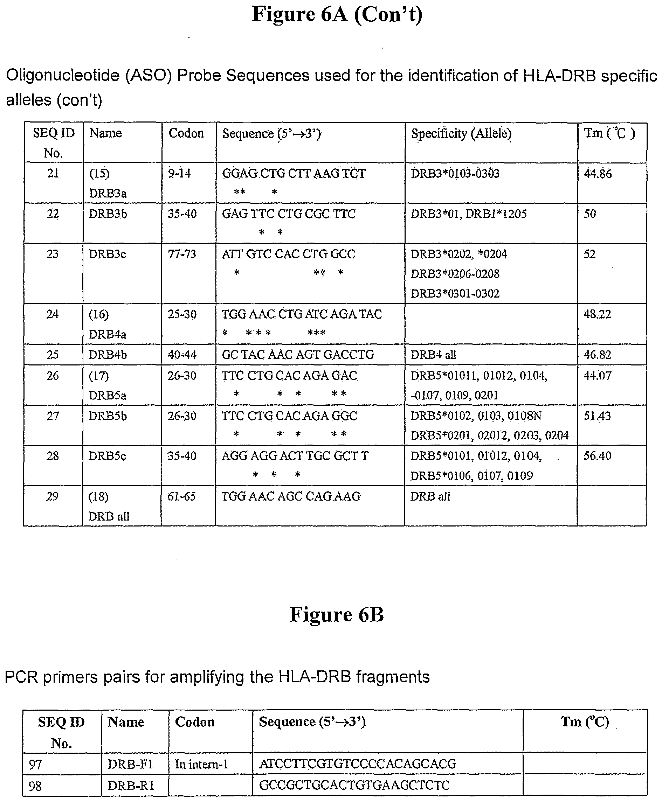

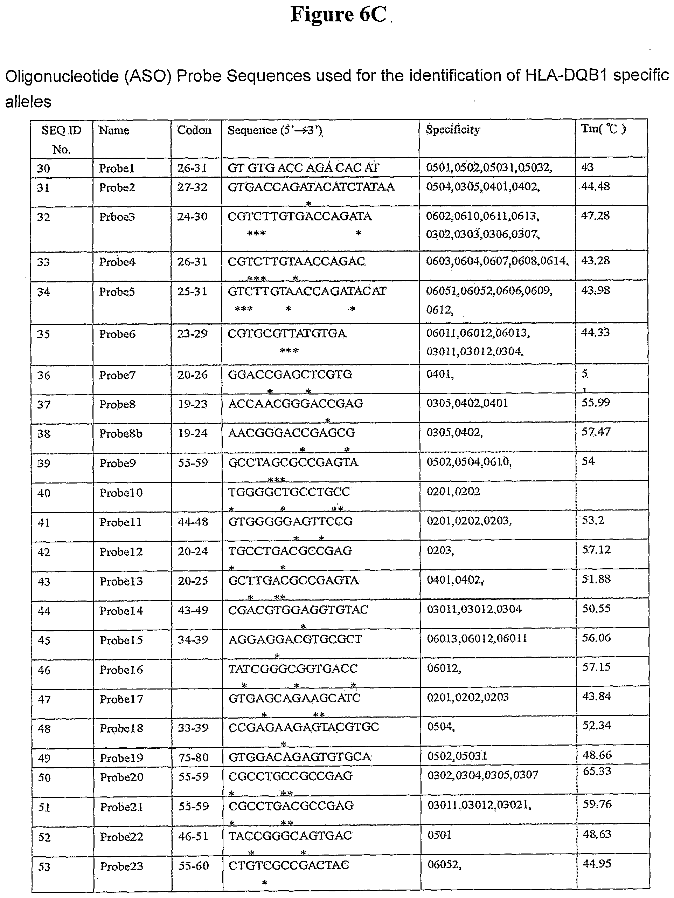

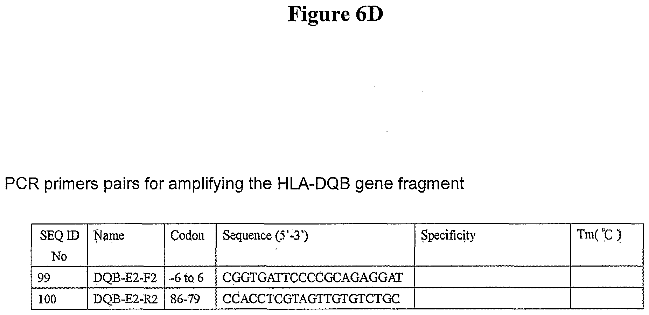

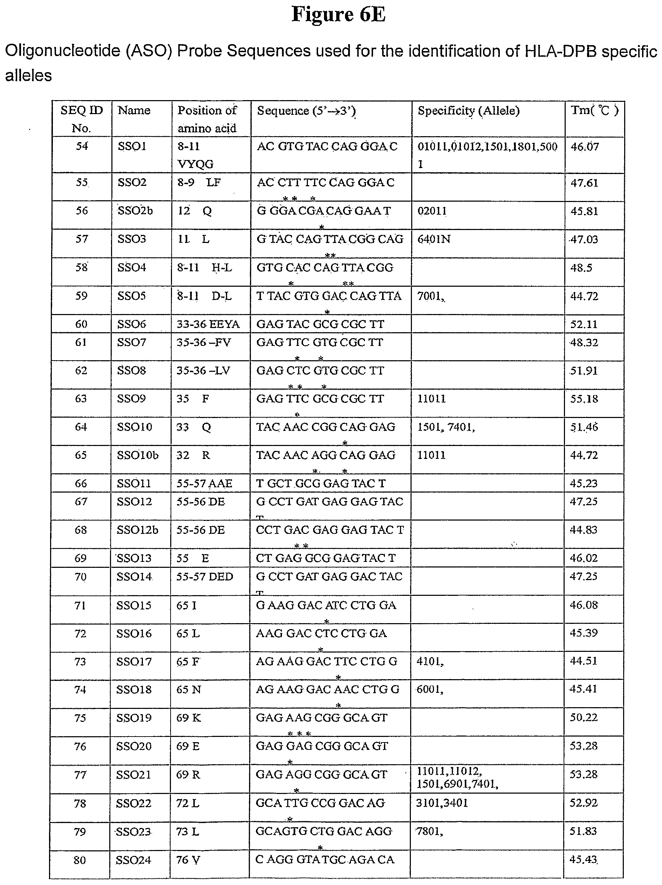

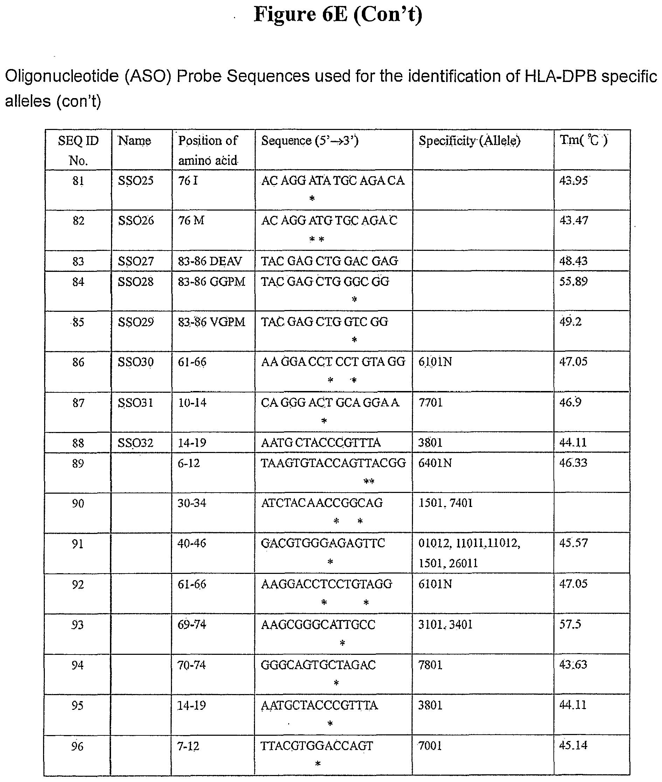

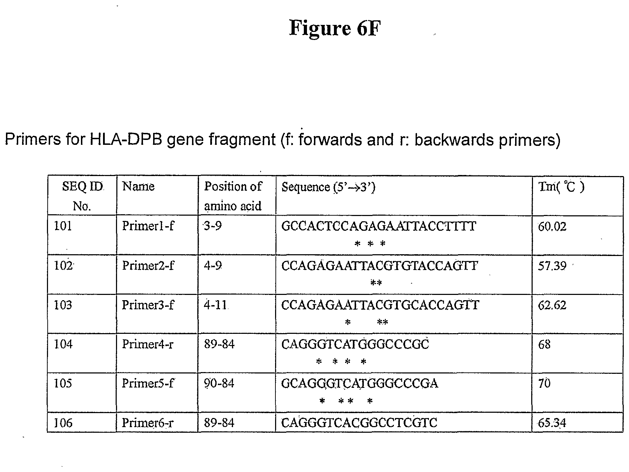

The primers and oligo-probes shown in Figure 6 have been tested and confirmed to be useful for the classification of HLA genotypes corresponding to the DR, DB and DP genes reported above. Following the scheme presented in Figure 1, additional primers and/or oligo-probes can be obtained, tested and validated for a more comprehensive genotyping. Although, in the data validating examples, PCR was used for amplification, any method that can produce specific target sequence(s) in sufficient quantity for the ASO-RDB flow-through hybridization analysis may be used. Other appropriate amplification methods or technique is readily apparent to one of ordinary skill in the art reading the teaching herein. Amplification may not be necessary if sufficient quantity of the target sequence(s) can be obtained for the ASO-RDB flow-through hybridization analysis. Detection can be accomplished by labeling of the target DNA or conjugates.

Although HLA genotyping is exemplified in this application, the Single nucleotide polymorphism (SNP)-based genotyping technique can be applied to other genetic materials and/or sequences obtained from any organism following the teaching of this application, such as the procedures shown in Figure 1. A flow-through device similar to those described in the U.S. Patent No. 5,741,547 or 6,020,187, or any new embodiments capable of carrying out flow-through hybridization process can be used.

Other than for DNA fingerprinting, SNP genotyping can be utilized for identification of gene fragments, or polymorphism of genes that have altered or attenuated the function of the gene in question. For this reason, the present invention can be used for rapid, definitive identification of infectious agents, inherited disease caused by the specific DNA sequences, or the presence or absence of such infectious agents or DNA sequences that cause inherited diseases.

The present invention provides a method of performing rapid nucleic acid detection, comprising the steps of: (a) obtaining a sample comprising a target nucleic acid molecule; (b) mixing the target nucleic acid molecule with (i) a first probe that will bind to the nucleic acid molecule, and (ii) a second agent that will bind to the first probe, thereby forming a nucleic acid molecule complex in solution, wherein the nucleic acid molecule complex comprises the first probe and the second agent, the second agent comprising a signal generating labeling tag or an enzyme-linked conjugate; (c) applying in a flow through manner the solution comprising the nucleic acid molecule complex to an array comprising a third probe that will bind to the complex, thereby capturing the nucleic acid molecule complex on the array; and (d) detecting the captured nucleic acid molecule on the array.

The present invention also provides a device capable of practicing the detection methods described above.

The present invention also provides a lateral flow hybridization device for detecting target analytes of nucleic acid molecules in a solution, the device comprising: (a) a membrane for immobilizing capture molecules capable of capturing target analytes, wherein the membrane is positioned in a reaction chamber where it can be maintained in controlled temperature conditions; (b) controlling elements that can be regulated to maintain the reaction chamber in the controlled temperature conditions; (c) connecting elements for connection to a power supply and control unit that can regulate and maintain the controlled temperature conditions; and (d) liquid delivery elements capable of accepting and removing solution to and from the reaction chamber, wherein the device is adapted such that, in use, the solution is maintained in a lateral flow direction that allows the solution flows through the membrane pores in cross-section so that all target analytes pass through the capture molecules immobilized on the membrane, thereby providing higher sensitivity of analytes detection; characterized in that the reaction chamber comprises a heat conduction plate which is in contact with heating and cooling elements, wherein the heat conduction plate is adapted to be in contact with detection membrane strips on the membrane during use when the target analytes flow laterally through the membrane.

The present invention also provides a lateral flow hybridization system comprising a plurality of the hybridization devices described herein, wherein each of the hybridization devices are connected to a power supply and control unit capable of supplying energy and providing regulatory control to the hybridization devices.

The present invention also provides a lateral flow hybridization device comprising: (a) more than one reaction chambers, each of which comprises a membrane for immobilizing capture molecules capable of capturing target analysts; (b) controlling elements that can be regulated to maintain the reaction chamber in controlled conditions; (c) connecting elements for connection to a power supply and control unit that can regulate and maintain the controlled conditions; and (d) liquid delivery elements capable of accepting and removing solution to and from the reaction chambers, wherein the solution is maintained in a lateral flow direction that allows the solution flows through the membrane pores in cross-section so that all target analysts pass through the capture molecules immobilized on the membrane, thereby providing higher sensitivity of analysts detection.

### DETAILED DESCRIPTION OF THE DRAWINGS

**Figure 1A** shows a method of the present invention for constructing an ASO probe and PCR primer database for any intended target(s) for which analysis is to be done.
**Figure 1B** shows a method of the present invention for building an Oligo-Probe Library for HLA Genotyping and any intended target(s) for which analysis is to be done.
**Figure 1C** shows a method of the present invention for selecting genetically unlinked target sequence(s) screen and design primers and SNPs for DNA fingerprinting identification.

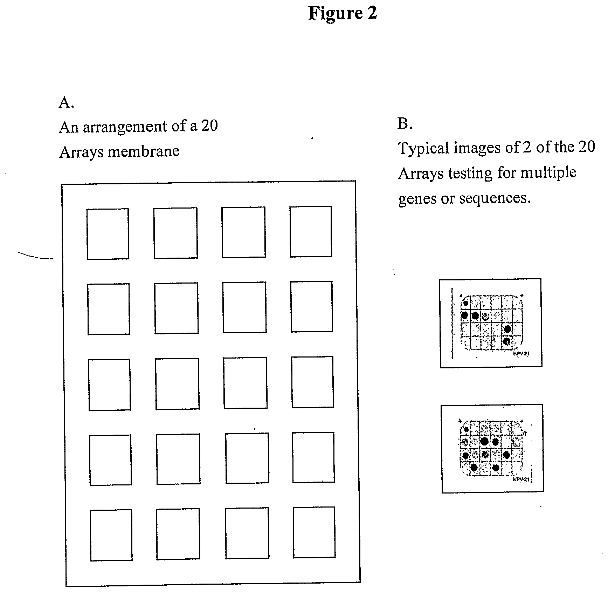

**Figure 2** shows a membrane of the present invention designed for high throughput analyses.

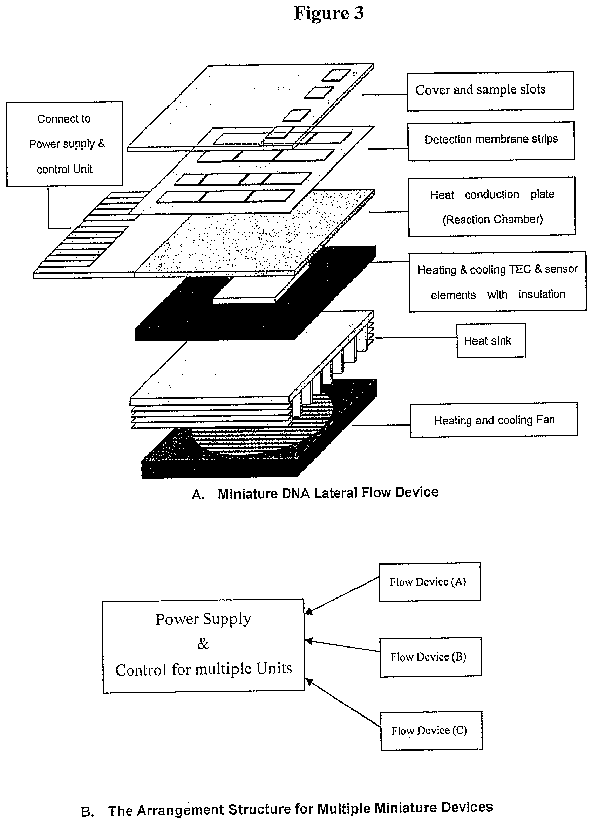

**Figure 3** shows an exploded view of a hybridization device of the present invention, and arrangements of multiple lateral flow-through detection devices connected to a central control unit. In an embodiment, the hybridization device comprises a central controlling unit connected to one or more lateral flow device. The central controlling unit provides power to and controls the lateral flow device where the hybridization process and developing procedures are carried out. Several reactions (or several samples and/or analytes) can be tested simultaneously in a single lateral flow device or in several devices (controlled individually at different conditions) at the same time. The lateral flow device can be in a format of n x m dot matrix (array) or in the form of linear arrays (as shown). Since, during the reaction process, the test solution flows from one end of the array to the other end of the array (i.e., in an east to west, or in a north to south direction), the sensitivity of the detection is increased substantially. The extent of increase in sensitivity depends on the ratio of the total area of the membrane to the area of the dot or line containing the capturing probes. For example, assuming the total area of membrane is 100mm square, and the dot size is 1mm square. In a direct flow-through process (i.e., the solution flows from top surface through the membrane down to the other side of the membrane as in a conventional flow-through process), only 1/100 of the total test solution used will flow through the dot, the location where the target molecule will bind to the probe(s) immobilized on the membrane. However, if a lateral flow-through process is used, the sensitivity is only dependent on the ratio of the width of the dot to the width of the membrane (i.e., the cross section of the membrane). For instance, in a lateral flow-through process, the total amount of solution that will pass through a 1 mm dot provided on a 10mm x 10mm membrane will be about 1/10, which represents a 10-fold increase in sensitivity using the same amount of target analyte (test solution containing the target molecules). When a line array format is used in the lateral flow-through process, the sensitivity will be further increased since all the target molecules will pass through the line extending across the strip (or membrane). The lateral flow-through process allows quantitative measurements to be taken during the hybridization process because the flow of the analyst is more uniform.
**Figure 4** shows a HLA-DPB1 genotyping results based on samples obtained from a Chinese population.
**Figure 5** shows HLA-DPB1 allele and genotype frequencies for a Chinese population.
**Figures 6A-F** show the ASO Oligonucleotide probe sequences identifying the respective HLA genotypes, and the PCR primer sequence pairs for amplify the corresponding fragments for analysis.
**Figure 7** shows examples of Low-density (Macro) Arrays containing different controls (internal and reference).

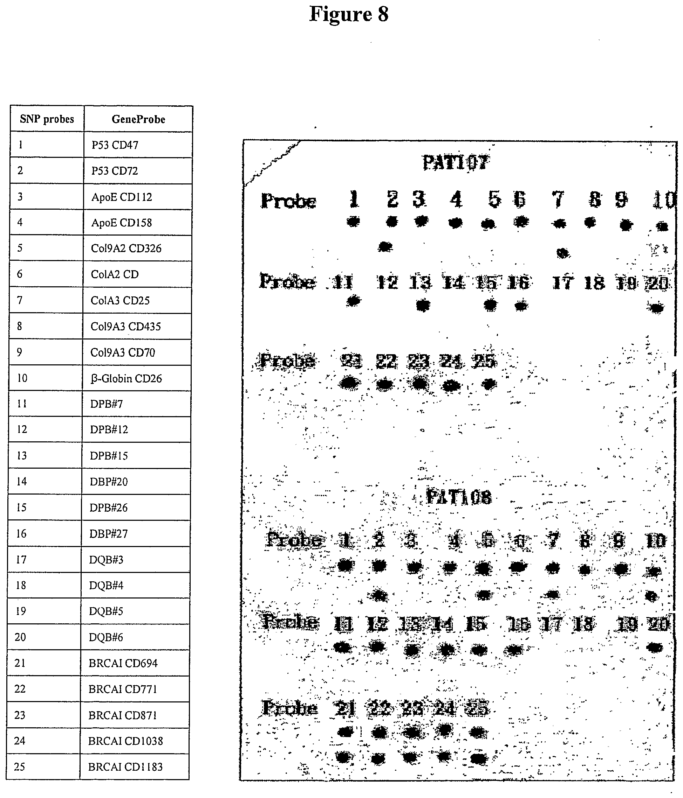

**Figure 8** shows a set of marker genes used for SNP DNA Fingerprinting and two of the SNP imaging profiles for given individuals that can be positively excluded one another.
**Figure 9** shows typical examples of Array images for genotyping HPV viruses in which the most common 23 separate types and a generic probe for detecting the other rare types if present.
**Figure 10** shows an example of an Array Format for detecting upper respiratory tract infectious viral pathogens.

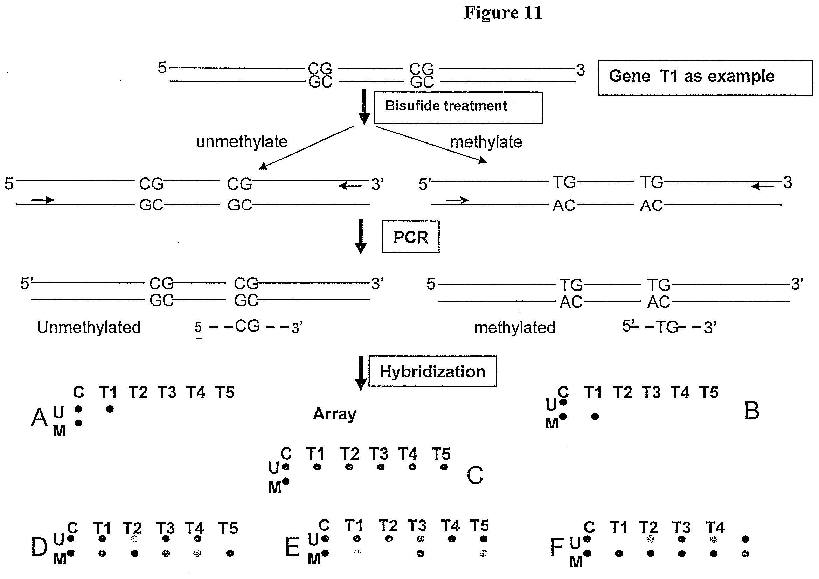

**Figure 11** shows a method for performing different extent of methylation on the CpG islands; the Methylation Specific PCR and Flow-through Hybridization assay. The presence of methylation on the DNA strands can be detected by disulfide modification by which the CmG will be changed into TG whereas unmethylated CG stays unchanged. Hence the ratio of TG to CG of a given CG islands can either be determined by real time quantitative methylation specfic PCR (MS-PCR or QMS-PCR) or by co-amplification followed by hybridization with methylation-specific probe and unmethylation-specific probe as described herein. After amplification, unmethylated strand will be captured and seen only with the unmethylated probe as shown in Array A. Methylated strand will be seen at Array B. In cases where all genes studied are not methylated, none of the methylated dots will show any signal as in Array C. On the other hand, various profiles will be seen corresponding to different extent of methylation in individual genes. If proper set of genes are selected, one can probably relate differences in intensity profiles to certain diseases such as cancers.

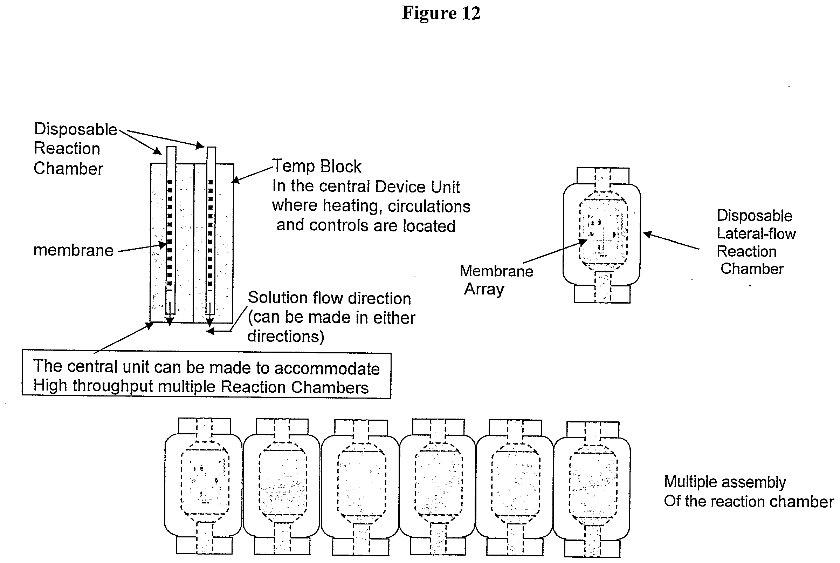

**Figure 12** shows a Lateral Flow Reaction Chamber Assembly as described herein. The disposable single unit or multiple-assembly has all the advantages of the lateral flow fast testing used in immunochemical testing strips. When incorporated into the control unit it will achieve the most stringent conditions for sensitive and specific nucleic acids analysis in a fraction of time of any conventional methods in use.
**Figure 13** shows a scheme for one-step hybridization.
**Figure 14** shows a comparison of conventional hybridization and the single-step signal amplification flow-through hybridization detection assay described herein.
**Figure 15** shows primers and probe sequences for respiratory tract pathogens. All sequences except those for Metapneumovirus were reported by Grondahl et al., J. Clinical Microbiology 37:1-7 (1999). Sequences for Metapneumovirus were reported by Mackay et al., J. Clinical Microbiology 41:100-105 (2003).
**Figure 16** shows examples of molecular markers of cancer.
**Figure 17** shows a partial list of genes responsible for regulation of metabolic pathways leading to cell homeostasis.
**Figure 18** shows examples of methylation specific primers and probes sequences.
**Figure 19** shows candidate genes for hypermethylation assays.

The present invention will be described in connection with preferred embodiments, however, it will be understood that this is not intended to limit the invention to the embodiments described. On the contrary, the intent is to cover all alternatives, modifications, and equivalents as may be included within the spirit and scope of the invention as defined by the appended claims.

### DETAILED DESCRIPTION OF THE INVENTION

### I. Genotyping

The following describes a method of the present invention for obtaining the HLA genotyping ASO probes and PCR primers from genomic sequence database, and for performing HLA genotyping analysis:

### 1. Select appropriate gene segments and determine appropriate PCR primers and ASO sites

Select appropriate target sequence(s) to be analyzed by screening data from GenBank and/or by performing population screening by sequencing target genes or DNA segments to obtain SNP or ASO profile appropriate for HLA genotyping.

A method of the present invention for selecting appropriate target sequence(s) is detailed below:

Find all the HLA DNA sequence data from GenBank according to their individual class (i.e., Class I or Class II) and subtypes (i.e., DR or DQ or DP, etc.). Align HLA DNA sequences based on individual classes and subtypes to determine the most polymorphic region(s) that will be appropriate for use in HLA genotyping.

Within polymorphic region(s), identify PCR primer pair(s) that is conserved among the subtypes of interest so that when using these primers, the PCR products can always be amplified for all the subtypes of interest for further analysis using the flow-through hybridization process.

To validate the usefulness of these primers, PCR amplification is performed using a large number of random samples to obtain statistically satisfactory data for positive amplification of the region(s) in question, and to ensure that any drop out of any allele does not result due to false negative amplification process. In addition to statistical validation, internal control(IC) is included to ensure that an inhibitor of PCR is not present in the test sample which will prevent the PCR amplification reaction.

The design of IC (may be 1 or more) will depend on the test required. In the case of HLA and SNP fingerprinting, and genotyping of genes responsible for genetic diseases, the nature of IC sequence will be non-homologous to the human genome in question (i.e., a sequence totally unrelated to sequence in question). Since human cells are mostly diploid (except germline) genome, the concentration issue is much simpler because theoretically the concentration of the test loci should be either 1 (homozygous) or 0.5 (heterozygous). Hence, two different sequence fragments flanked by the same PCR primer sequences can be used for IC. This means that using a fragment different from that of the target fragments to be analyzed, one can use loci of known character of homozygous and heterozygous sequences within the fragment (that is two different probes: 1 homo; 1 hetero in the fragment) as probes so that these IC concentration ratio can be calculated. Since the PCR amplification does not affect the relative concentration, the ratio of signal will be 2:1 theoretically. Any fluctuation will be seen as the hybridization efficiency. The two ICs with a ratio of 2:1 serves as the reference for homozygous and heterozygous, respectively, for the loci in question. If the observed result indicates only one allele, and the concentration in question is equal to or less than 0.5 of the homozygous IC or close to 1 compare to the heterozygous IC, the possibility of loss of heterozygousity should be investigated, meaning: may be one of the two chromosomes has lost or could not amplify. The expected result of ONE dot is homozygous which indicate the two chromosomes are the same, and therefore the concentration should be closer to the homozygous IC than that of the heterozygous IC. Hence, ICs will be important for obtaining accurate results since clinically dependable result is crucial for any diagnostic test.

Additional information on DNA data bank and alignments can be found in the reference section of this application (see Reference No. 5-6).

The procedures described herein for HLA genotyping can also be adopted by one of ordinary skill in the art reading the teaching herein for genotyping of other genes or DNA sequences of interest, or for determining SNP profiles for DNA fingerprinting/identification process performed in combination with flow-through hybridization process.

To maximize the efficiency of PCR amplification, the fragment length of the ASO probes is normally selected or kept as short as possible, preferably to within a few hundred base-pairs. If suitable primer pairs cannot be identified, adjustments on the components in the PCR reaction mixtures and the PCR processes, i.e. the PCR program, can be made to optimize the amplification reaction to ensure the production of amplicons during development of the genotyping process. Such development processes are apparent to persons of ordinary skill in the art after reading the teaching herein. In some cases a totally conserved sequences for PCR Primer cannot be found and degenerate primers or multiple primers for the same loci may have to be use. In these cases, appropriate adjustments to the PCR conditions are made to ensure that all loci will not be missed.

When unique region(s) for successful subtype differentiation cannot be identified, multiplex PCR may be required. The primer pairs are designed to Tm values within the operable range for successful annealing during the amplification process. As used herein, "Tm" means, for example, the temperature of the reaction where the concentration of DNA molecules in double strand as well as single strand is equal. Hence, at higher temperature more double strand DNA will become single strand, and conversely at lower temperature, more single strand molecule will anneal into double strand. For a given population where sequence data is not available, a population screening by direct DNA sequencing is usually performed. For example, a screening of a sample Chinese population was performed as follows: (a) Random samples of over a hundred subjects were amplified by PCR using two pairs of primers, followed by hybridization using the ASO Probes designed based the procedures described in the following sections; (b) The results were confirmed by DNA sequencing.

Using the data obtained above, the ASO sites to be used for genotyping are determined and selected. Determine whether the sites selected are indeed unique for HLA typing within a population using data obtained from GenBank or generated by sequencing random samples. The procedures for designing ASO probes and for validating the uniqueness of the probes are as follows:

From the selected region(s) of the alignments for which satisfactory polymorphism among the subtypes are found, further search for a unique 20-30 nucleotide sequence or fragment is performed. The unique sequence or fragment is used as an allele specific oligonucleotide (ASO) probe to capture the amplified target by hybridization process in order to detect the presence of such unique HLA subtype. As used herein, "unique sequence or fragment" means, for example, that the sequence or fragment is completely homologous to only one sequence, among these subtype sequences.

To verify that the ASO probe(s) is unique, the sequence is matched with all human DNA sequence data which are available in the GenBank or in its European equivalent to determine if indeed the ASO probe has 100% match with ONLY the HLA subtype of interest. This will ensure that at least (until new sequences are discovered) among the available data, the ASO probe(s) is unique for a given region.

Since PCR fragment length is kept short to improve the efficiency of amplification, one ASO probe may not be sufficient to provide a definitive differentiation to determine the unique subtype. Hence, a set of multiple ASO probes within one PCR fragment and/or in different fragments using the multiplex PCR may have to be used to give a definitive genotyping classification. In this case, a specific pattern of the ASO array will be generated by the hybridization process for each of the given HLA subtypes. After thorough analysis of the data, the ASO sequences undergo validation using random human DNA samples.

Similar procedures can be used for other genes and genetic materials from other organisms. The primer sequences and the number of ASO probes vary for different genes and for different applications. For example, human identification may require a 50 or more ASO array to obtain definitive identification. The detection format can include an array of dots or linear lines depending on the configuration of the flow-through hybridization device.

### 2. Performing ASO-RDB detection

ASO oligonucleotides are immobilized on a membrane or any suitable matrix for capturing the target loci. As used herein, "membrane" means, for example, any suitable matrix material capable of immobilizing ASO oligonucleotide probe(s) and porous enough for test solution containing the target nucleic acid molecules to pass through freely. In an embodiment, the membrane or matrix may be constructed of Nylon, NC, Biodyne, Porex, and porous metal or durable gel matrixes.

Immobilization of the target sequence (or loci) can be achieved by covalent bonding, non-covalent (i.e., electrostatic, hydrophobic or any other interactions including UV cross-linking) interactions, or interactions through mediators such as receptors or antibodies. Avidin-biotin linkage or ASO poly-T tailing for UV cross-linking is equally effective.

Target sequences are amplified using appropriate primers to generate enough amplicons to enable adequate analysis. The target molecules can be appropriately labeled for signal generation according to the method of signal detection used for final analysis. Labeling can be performed on one or both of the primers by covalent linkage of the label molecule at the 5'end or using one of the four dNTP labeled nucleotides during the PCR amplification process for extension labeling of the newly generated amplicons. The label molecule can be of any kind as long as the signal can be monitored and developed. Biotin coupled with avidin-enyzme conjugate can be used for color detection. Other suitable labeling systems include, but are not limited to, colloidal gold conjugate and fluorescence label, magnetic particles conjugate, quantum dots, chemiluminescence label molecules, or other suitable systems already developed or to be developed may be used as well.

ASO profile analyses are performed using flow-through hybridization process such as the method described in U.S. patent number 5,741,647. Hybridization is performed in the hybridization chamber with ASO capture probes coupled to the membrane. An ASO profile analysis is described below:
(a) Denaturing and contacting solution containing target DNA or sequence with membrane;
(b) Washing membrane with washing (or SSC or blocking) solution, preferably three times; and
(c) Developing color for visual inspection or spectrometric measurements. For quantitative measurements, one can use a scanner and imaging software to perform the analysis. Alternatively, target DNA or sequence can be labeled with fluorescence dye and analyzed using a spectrometric imager immediately after the washing step.

The results are compared with known sequence data to ensure accuracy of genotyping assay. Probes and testing conditions can be further modified to improve the accuracy of genotyping assay, and RDB-ASO data is verified by DNA sequencing.

### 3. Validation

Validation is performed using random samples. A validation procedure of the present invention is described below:

As mentioned above, once the primers pair(s) and ASO capturing probe (s) are immobilized on a membrane, random DNA samples of sufficient number are used to perform PCR amplification. Hybridization of target sequence(s) or amplified and labeled DNA products or molecules is performed to generate an ASO array pattern.

HLA subtype and corresponding DNA sequence(s) are determined from ASO probes. Corresponding PCR samples are prepared for direct DNA sequencing.

Agreement of the results obtained from DNA sequencing and from flow-through array is needed to validate the HLA genotyping assay. Samples for validating genotyping assay can be obtained from any randomly selected individuals. Once the samples have been obtained, true genotyping can be performed by DNA sequencing as stated above. If sequencing data agree with those obtained from flow-through array, the validity of the data is confirmed. Further validation of genotyping assay can be performed through field tests, such as testing random samples, analyzing data and comparing statistically values such as sensitivity, specificity, positive predicted value or negative predicted value, at independent laboratories to evaluate the accuracy of genotyping assay.

### II. SNP Genotyping

The following is a method of the present invention for constructing a SNP database and developing a SNP genotyping assay:

Select SNP sites and determine the power of exclusion. As used herein, "power of exclusion" means, for example, the accuracy of the method in differentiating people or organisms. For instance, power of exclusion of 1 in 10 billion or 100 billion means that when using a selected number of SNP sites one can only expect to find two identical individuals in screening 10 or 100 billion individuals, respectively.

Select appropriate SNP oligonucleotide probes for capturing specific target sequences to be analyzed either by screening data from GenBank or by performing population screening, i.e., direct DNA sequencing of target genes or target DNA segments, to obtain SNP profile and population frequencies.

From these data, identify the SNP sites to be used for fingerprinting based on polymorphic frequency, and determine whether or not the selected sites are indeed hot spots for mutation within a population by sequencing the samples obtained from random population screening.

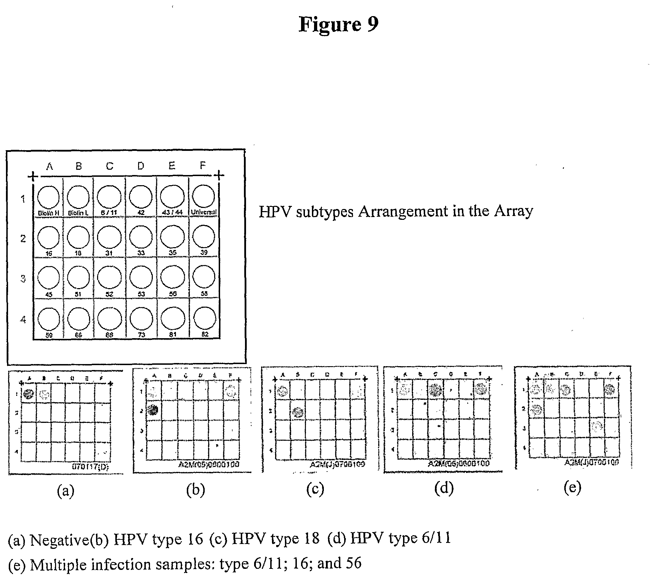

Determine the number of SNP probes required, and calculate total heterozygosity to determine the power of exclusion with the given number of SNP points used for the analysis profile pattern (i.e., the SNP dot array as shown in Fig 9).

Power of exclusion depends on the number of SNP in a given loci. For example, in a given loci, if 2 different bases, e.g., G and A, are found in a sample population at 50% each. The probability is 1/2. If 50 of such sites can be found (these sites are randomly distributed and are not linked to each other in the chromosome) the differentiation power will be 1/2 to the power of 50.

Perform SNP profile pattern (a combination of SNP array showing the array of corresponding genotypes in each loci of that individual) detection.

Design appropriate primers for amplification, and select appropriate SNP-probes for hybridization detection after the loci are screened and selected as described above.

Amplify target sequence(s) and perform SNP profile analyses using flow-through hybridization process such as the method described in U.S. Pat. No. 5,741,647.

Compare the data obtained with known sequence data to evaluate the accuracy of the SNP genotyping analysis.

Modify SNP probes and testing conditions to improve the accuracy of the SNP genotyping analysis. RDB SNP data are verified by DNA sequencing.

It should be understood that under the general term of SNP genotyping analysis, the invention described herein should not be considered limited to human genome; nor the SNP be limited to Single Nucleotide Polymorphisms (or single base mutations). The term "SNP" shall include short deletions and insertion of nucleotides. "Genotyping" shall apply to any genetic sequences of any organism where variation of sequences would enable any skilled professional in the art to differentiate the variation among different sequences in the case of nucleic acids and variation of structures

### III. Hybridization And Signal Development

Conventional hybridization involves many solution changes and transfers steps and therefore is a labor intensive and time consuming process. The Flow-through inventions disclosed in U.S. Patent Nos. 5,471,547 and 6,020,187 eliminate all transfer steps and substantially reduce the reagents volume to make the hybridization a very efficient process. Nonetheless, the number of steps in the hybridization protocol remained basically similar. The present invention describes further significant improvements in the hybridization protocols which include:

Elimination of the pre-hybridization step in which the blocking and hybridization steps are combined using an improved reagent mixture (i.e., DNA samples are placed in hybridization solution and flow-through detection membrane without pre-hybridization).

A single-step hybridization process in which the target sequences or molecules are labeled with fluorescence tags, quantum dot, colloidal gold particles, magnetic particles or other appropriate labeling tags to eliminate the enzyme-link conjugate substrate color development step. These improvements will enable a technician to complete the entire hybridization process in 5 minutes or less. Hence, the method of the present invention should provide further savings in terms of time and reagent cost.

### IV. Lateral Flow-Through And Miniature Devices

New embodiments described herein take advantages of the physical dimensions of the relative length and area of the membrane used to capture the target molecules whereby increasing the sensitivity and lowering the detection limit to achieve the best results. Examples given below illustrate such principle. Other embodiments can be constructed to optimize for complete capture of the target molecules. Thus recirculation of the hybridization mixture is an alternative. This and automation process can be incorporated into the examples described herein.

The invention being generally described, will be more readily understood by reference to the following examples which are included merely for purposes of illustration of certain aspects and embodiments of the present invention, and are not intended to limit the invention.

The present invention provides a method of performing rapid nucleic acid detection, comprising the steps of: (a) obtaining a sample comprising a target nucleic acid molecule; (b) mixing the target nucleic acid molecule with (i) a first probe that will bind to the nucleic acid molecule, and (ii) a second agent that will bind to the first probe, thereby forming a nucleic acid molecule complex in solution; (c) applying in a flow through manner the solution comprising the nucleic acid molecule complex to an array comprising a third probe that will bind to the complex, thereby capturing the nucleic acid molecule complex on the array; and (d) detecting the captured nucleic acid molecule on the array.

In general, the target nucleic acid molecule is of human, bacterial, or viral origin. In one embodiment, the human is having or is suspected to have cancer. In another embodiment, the target nucleic acid molecule is obtained without amplification.

Preferably, the first probe and the third probe bind to different regions of the target nucleic acid molecule. In one embodiment, the third probe is an allelic-specific oligonucleotide probe. The allelic-specific oligonucleotide probe can be generated, for example, from nucleic acid sequence data bank. In another embodiment, the third probe can identify single nucleotide polymorphism of the target nucleic acid molecule. For the detection of the captured nucleic acid molecule, it can be generally performed by fluorescence tags, quantum dot labeling, colloidal gold particle labeling, magnetic particle labeling, or enzyme-linked substrate assay.

The present invention also provides a method of performing rapid nucleic acid detection, comprising the steps of: (a) obtaining a sample comprising a target nucleic acid molecule; (b) mixing the target nucleic acid molecule with (i) a first probe that will bind to the nucleic acid molecule, (ii) a first antibody, and (iii) a labeling agent, wherein the first probe will form a complex with the target nucleic acid molecule, and the first antibody will bind to the resulting complex; (c) applying in a flow through manner a solution comprising the nucleic acid molecule complex to an array comprising a second antibody that will bind to the first antibody, thereby capturing the nucleic acid molecule complex on the array; and (d) detecting the captured nucleic acid molecule on the array.

In general, the target nucleic acid molecule is of human, bacterial, or viral origin. In one embodiment, the human is having or is suspected to have cancer. In another embodiment, the target nucleic acid molecule is obtained without amplification. For the detection of the captured nucleic acid molecule, it can be generally performed by fluorescence tags, quantum dot labeling, colloidal gold particle labeling, magnetic particle labeling, or enzyme-linked substrate assay.

The present invention also provides a device capable of practicing the detection methods described above.

The present invention also provides a lateral flow hybridization device comprising: (a) a membrane for immobilizing capture molecules capable of capturing target analysts, wherein the membrane is positioned in an reaction chamber where it can be maintained in controlled conditions; (b) controlling elements that can be regulated to maintain the reaction chamber in the controlled conditions; (c) connecting elements for connection to a power supply and control unit that can regulate and maintain the controlled conditions; and (d) liquid delivery elements capable of accepting and removing solution to and from the reaction chamber, wherein the solution is maintained in a lateral flow direction that allows the solution flows through the membrane pores in cross-section so that all target analysts pass through the capture molecules immobilized on the membrane, thereby providing higher sensitivity of analysts detection.

In general, the power supply and control unit is capable of supplying energy and providing regulatory control to maintain the reaction chamber in the controlled conditions. In one embodiment, the controlled conditions comprise accurate control of temperature within 0.5 degree C.

In one embodiment, the controlling elements of the hybridization device are heating and cooling elements such as Peltier heating and cooling elements, or elements for circulating liquid or air, or any other means known in the art for controlling temperature for the reaction chamber. In another embodiment, the heating and cooling elements comprise heat conduction plate, heating and cooling sensor, heat sink, and heating and cooling fan.

In general, the membrane in the reaction chamber is formed of material containing nitrocellulose, nylon, Nytron, Biodyne, or Porex, or other porous materials known in the art.

In one embodiment, solution containing target analysts is delivered to the reaction chamber and the membrane by regulated liquid pumping. For example, liquid pumping can be used to recirculate solution containing the target analysts through the membrane.

The present invention also provides a lateral flow hybridization system comprising more than one of the hybridization devices described herein, wherein each of the hybridization devices is connected to a power supply and control unit capable of supplying energy and providing regulatory control to the hybridization devices. Hence, in one embodiment, each of the hybridization devices can be controlled independently by the power supply and control unit so that each hybridization device can perform different analysis under different conditions.

The present invention also provides a lateral flow hybridization device comprising: (a) more than one reaction chambers, each of which comprises a membrane for immobilizing capture molecules capable of capturing target analysts; (b) controlling elements that can be regulated to maintain the reaction chamber in controlled conditions; (c) connecting elements for connection to a power supply and control unit that can regulate and maintain the controlled conditions; and (d) liquid delivery elements capable of accepting and removing solution to and from the reaction chambers, wherein the solution is maintained in a lateral flow direction that allows the solution flows through the membrane pores in cross-section so that all target analysts pass through the capture molecules immobilized on the membrane, thereby providing higher sensitivity of analysts detection. General features for the membrane, controlling elements, and liquid delivery have been described above. In one embodiment, the reaction chambers of this hybridization device are disposable. In another embodiment, each of the reaction chambers is a separate unit.

### EXAMPLE 1

The following procedures were used to determine HLA genotypes of 140 human samples obtained from a sample Chinese population. The results were used to develop a kit for rapid classification of HLA subtypes of the first 2 digit codes corresponding to WHO nomenclature. In one embodiment, a kit can be designed to cover HLA-DR, -DQ and -DP alleles which are typically used for tissue matching between a donor and a recipient prior to organ transplantation.

### Test Procedures:

### A. Isolation of DNA

The following protocols are recommended, but alternative procedures which are readily apparent to one of ordinary skill in the art and which are equally effective may be employed. Nucleated cells such as WBC or tissues are washed with PBS, centrifuged, and the supernatant is removed. The pellet is re-suspended in 200 µl PBS. DNA extraction is performed with QIAamp DNA mini kit (QIAGEN) following Blood and Body Fluid Spin protocol as recommended by the manufacturer. Other commercially available kits for isolating DNA may also be used. However, DNA isolation procedures that produce purified DNA that do not contain DNA polymerase inhibitor are crucial to ensure efficient amplification. Elute DNA in 50-200µl Buffer AE and store at -20°C until use.

### B. PCR amplification

Since PCR is extremely sensitive process, special care must be taken to prevent cross contamination and/or to prevent false positive results. Hence, the following guidelines should be observed: Wear surgical gloves at ALL TIMES when performing PCR procedures. Preferably, prepare PCR reaction mixtures in a pre-PCR area or a clean PCR preparation station where no amplification products are present. PCR reaction and hybridization should be performed in separate areas. For example, hybridization should be performed in a post-PCR area.

Use 70% ethanol and paper towel to clean the working bench/area. Use 70% ethanol and paper towel to clean all the pipettes before starting PCR amplification. Use filtered/sterile tips for ALL pipetting steps. NEVER reuse any tips.

PCR is just one of the many techniques which can be used for amplification. Other amplification techniques, which are readily apparent to one of ordinary skill in the art, such as various methods for isothermal amplification using appropriate primers to amplify target sequence(s) to obtain sufficient quantity of target sequence(s) (or DNA molecules) to carry out flow-through array detection may be used.

PCR reactions are performed using, for example, commercially available polymerase AmpliTaqTM Gold (Applied Biosystem). Five primers pairs (1 pair used for amplifying the DR genes, i.e., forward primer DRB-F1: 5'-ATCCTTCGTGTCCCCACAGCACG-3' (SEQ ID No. 97) and the reversed primer DRB-R1: 5'-GCCGCTGCACTGTGAAGCTCTC-3' (SEQ ID No. 98); 1 pair used for amplifying the DQ genes, i.e., the forward primer is DQB-E2-F2: 5'CGGTGATTCCCCGCAGAGGAT-3' (SEQ ID No. 99) and the reversed primer is DQB-E2-R2: 5'-CCACCTCGTAGTTGTGTCTGC-3' (SEQ ID No. 100); and 3 pairs used for amplifying the DP gene, the forward primers (SEQ ID NOs. 101-103) and the reversed primers (SEQ ID NOs. 104-106) listed in Figures 6B, D and F (below) are individually 5'-end labelled with biotin. Other appropriate labelling methods described above can also be used. In 25 µl reactions, prepare a PCR master mix as follows (This example is intended to illustrate one particular program for PCR amplification reaction of the present invention. If other SNP primer sequence (s) are used, the condition for PCR and hybridization must be optimized):

| | Each reaction (µl) | 10 reactions |
|---|---|---|
| PCR-Mix | 19.00 | 190.0 |
| DNA Taq Polymerase | 1ul (1 unit) | 10ul (10 units) |
| DNA Template | 5 (~100ng) | - |
| Total | 25 | 200 |

Amplification program was optimized using PE 9700 thermal cycler. For PE 9700 (or MJ thermal cycler):

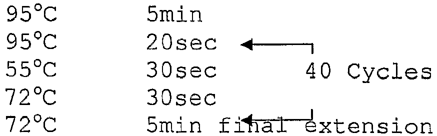

When using different primers or other thermal cycler, modifications to the cycling program may be required.

### C. Quality control for PCR

It is important to have a positive control and a negative control in every PCR analysis. Positive control is needed to demonstrate the efficiency and specificity of PCR, and negative control is needed to determine whether PCR reagents are contaminated. Internal controls are also needed to provide proper interpretation, and to ensure the dependability and accuracy of the data. The type and the number of IC depend on the type or nature of the test needed.

IC can be used to track each step of the hybridization reaction or procedure. For example, IC can be used to determine whether a sample was added or whether any inhibitors are present in a sample which will prevent PCR reaction from working properly. IC can also be used to track the efficiency of the reaction, or to determine the concentration of target molecule in a semi-quantitative or quantitative manner.

If a quantitative or semi-quantitative measurement is needed, the detection array or membrane can be prepared with a signal generating internal control (IC) onto the membrane to provide a detectable signal at predetermined concentrations when a preprogrammed or predetermined condition has been met. In such embodiment, the IC is developed simultaneously with the test samples after hybridization. In another embodiment, additional IC can be added into the PCR reaction mix to indicate the intrinsic PCR efficiency. This can serve also as an internal control to indicate the presence or absence of inhibitor within the PCR reaction.

The IC system in the PCR reaction mix of the present invention provides several advantages. Since the PCR reaction is carried out in the same reaction tube, the absence of IC and test sample signal indicates the presence of an inhibitor. If the same primer sequences for IC and test samples are used for the PCR amplification, it can serve as an intrinsic control for PCR reaction efficiency. IC can also be used to determine the detection limits or cut off value of the PCR reaction and/or the efficiency of the hybridization process, and to provide an indication of whether hybridization was successfully completed. In addition, IC can be used to determine whether reagents for signal development are acceptable or prepared properly, and whether proper procedures were followed during hybridization.

### D. Hybridization

### Preparations prior to hybridization:

Pre-warm hybridization solution (i.e., 2x SSC or any commercially available solution/product) to 42°C in a water bath before use. If precipitate is present in solution B (SSC + 0.5% SDS), dissolve the precipitate by incubating solution B at 42°C until the precipitate is dissolved. Keep the temperature at 42°C throughout the hybridization process to maintain set stringency.

Prepare NBT/BCIP working solution by dissolving a tablet in 10ml of solution C or PBS buffer (phosphate buffer saline). Protect the diluted working NBT/BCIP solution from light and store any unused solution at 4°C.

Equilibrate hybridization solution (2X SSC + 0.05% tween 20) to room temperature.

Denature all biotinylated PCR products by heating at 95°C for 5 min, and then chill on ice immediately for at least 2 min.

### Hybridization Set-up

For the flow through hybridization studies, one can use the direct flow through device described in U.S. Pat. No. 6,020,187 or a lateral flow device described in this application. For the genotyping of HLA in this example, a direct flow device is used. Switch on hybridization device to preheat at 42°C filled with distilled water.

Place the detection membrane(s) which are embedded with capture probe(s), such as those listed in the sequence listing below, in the hybridization chamber. Secure the membrane(s) with, for example, a cover lid.

### Hybridization of PCR products

When the temperature reaches 42°C (+/- 0.5°C), deliver 1ml of the pre-warmed hybridization solution for pre-hybridization to cover the membrane (NOTE: The temperature stated here is the optimal annealing temperature for the given probe sequence. In general, to skill in the art may design the probe sequence to fit this temperature. However if required, different temperature can be used to fit other sequences.) Incubate for at least 2 minutes with the cover closed to prevent heat loss during pre-hybridization.This is to ensure temperature equilibrium at the set temperature.

Add 0.5ml of the pre-warmed hybridization solution to each denatured PCR products separately for testing, and add the DNA samples into designated well. Contacting DNA samples which contain the target sequence(s) with membrane surface and incubate at 42°C for 5 minutes, and then allow DNA samples which to flow through the membrane. Hybridization is normally completed within 30 seconds. The 2-5 minutes incubation period ensures that the temperature of the DNA samples will reach the set temperature.

Wash the membrane with 3x 0.8 ml of hybridization solution.

### Color Development

Set temperature to 37°C. Start pump, and immediately begin dispensing 0.5 ml of blocking solution. Stop pump. Add another 0.5 ml of blocking solution and incubate for 5 min, then pump out the solution.

Turn off pump and add 0.5 ml of the enzyme conjugate. Let the membrane sit for 3 min. Color development works well between 25-37°C. Start pump. Wash the membrane thoroughly, preferably four times, with 0.8 ml of buffered saline solution at pH 7.4.

Turn off pump and add 0.5ml of the NBT/BCIP solution (from Roche). Cover lid. Incubate for about 5 minutes or until color develops. Note: DO NOT incubate for over 10 minutes.

Turn pump on to remove NBT/BCIP solution. Wash the membrane, preferably three to four times, with 1ml of solution B after the color has completely developed. Rinse membrane with 2 ml of demonized H₂O once.

Inspect results as soon as possible, preferably within 1 hour, by direct visualization, or scan the image for semi-quantitative detection.

### E. Result interpretation

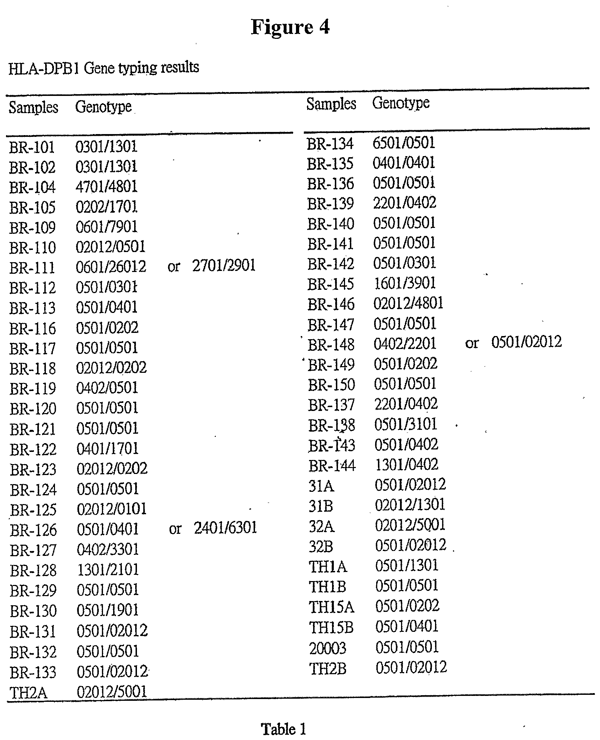

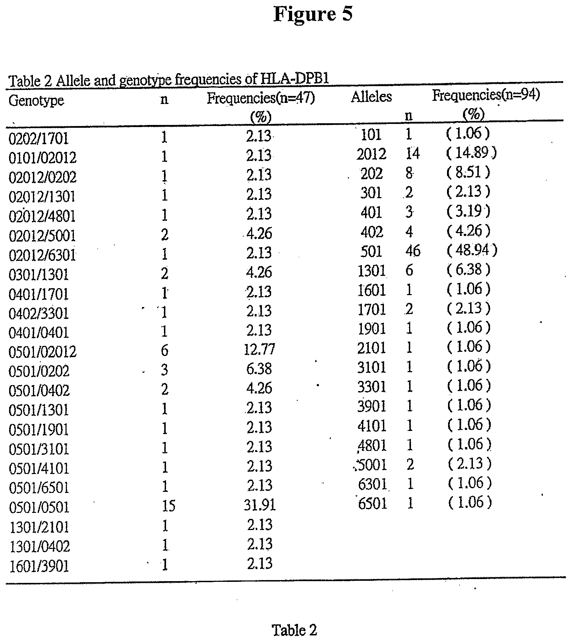

The presence of a clearly visible dot indicates a positive result. A total of 96 ASO probes (DRB: 29; DQB1: 24 and DPB1: 43), corresponding to SEQ ID 1-96 in the sequence listing for the HLA cluster, and five PCR primer pairs, corresponding to SEQ ID NO 97-106 in the sequence listing for amplification had been designed according to the scheme in Figure 1. The ASO probes and primer pairs have been evaluated and determined to be suitable for the classification of the HLA DR, DQ and DP genes. Genotypes and allele frequencies are given in Figures 4 and 5. All data obtained from reversed dot blot array by flow through hybridization (which used a total of 141 random human samples) were separately confirmed with DNA sequencing. In principle, any known ASO (or SNP) oligonucleotides of any organisms with adequate data to perform genetic analysis can be tested, identified or detected by the rapid, genotyping flow-through hybridization method of the present invention. Hybridization results are obtained within minutes, which translate into at least a ten-fold increase in speed over conventional hybridization techniques.

### EXAMPLE 2

### Simplified Genotyping Protocols and Devices

The flow-through DNA hybridization method and device as described in the U.S. patent Nos. 5,741,647 and 6,020,187, respectively, reduces hybridization time from many hours or days to minutes (the whole hybridization assay can be completed in 5-30 minutes depending the method used to generate detection signal). The device is also inexpensive to manufacture, and uses 10 times less reagents than convention hybridization devices which will lead to more affordable DNA diagnosis technology.

The present invention provides an inexpensive platform for studying the nucleic acids interactions using a low-density array format. This invention further provides a method of genotyping complex HLA systems. As illustrated above, the genotyping method of the present invention has been shown to provide significant improvements over conventional hybridization processes, even the hybridization process as described in U.S. patent Nos. 5,741,647 and 6,020,187.

The present invention further provides additional improvements over existing flow-through hybridization techniques. For example, the hybridization device of the present invention includes a detection membrane, such as the membrane shown in Fig 6 in a 4x6 array format. With the use of the ELISA 96 wells format, in which each well can be prepared with a 5-dot matrix array, for processing 96 samples and 5 different analysts to simultaneously. This increases the analysis throughput substantially.

The array format may be adapted by one of ordinary skill in the art to accommodate additional wells for analyzing a larger set of nucleotide sequences rapidly and inexpensively. The hybridization device of the present invention is a breakthrough in rapid DNA diagnostics. A lateral flow and miniature embodiment of the device of the present invention is depicted in Figure 3.

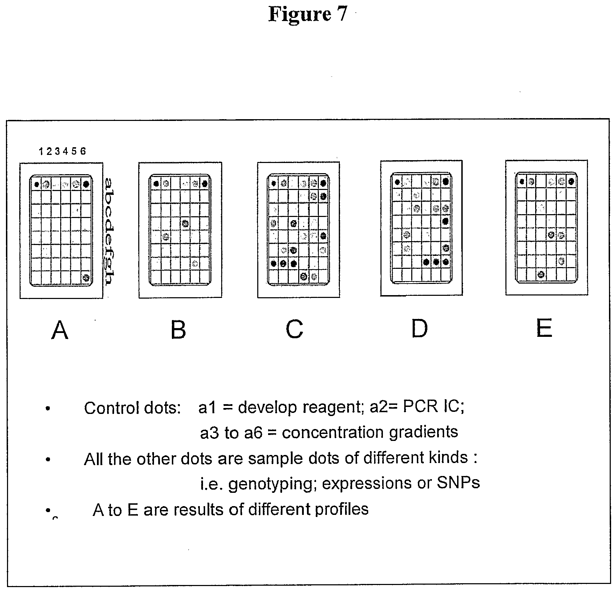

Significant improvements on this lateral device are described in Figure 3. The internal controls and concentration indicating dots are also described and depicted in Figure 7 by adding concentration gradient dots as part of the array to give the relative ranges on the detection results. As described, using the lateral flow procedure will significantly increase the sensitivity.

### EXAMPLE 3

### SNP Genotyping

To identify the DRB genotypes, the PCR were carried out with the primer pair of DRB-F1: 5'-ATCCTTCGTGTCCCCACAGCACG-3' (SEQ ID No. 97) and DRB-R1: 5'-GCCGCTGCACTGTGAAGCTCTC-3' (SEQ ID No. 98) and a 29 ASO probes (as listed in Figures 6A-B) were tested of which 18 were found to be best for the identification of the HLA-DRB alleles.

In the case of DQB1 genotypes, PCR is carried out using DQB-E2-F2: 5'-CGGTGATTCCCCGCAGAGGAT-3' (SEQ ID No. 99) and DQB-E2-R2: 5'-CCACCTCGTAGTTGTGTCTGC-3' (SEQ ID No. 100) as primers (see Figure 6D) which are able to generate a 260bp. The 24 SSO probes are used as capture probes for this DQB1 classification during hybridization.

To identify DPB1 genotypes, a total-of 43 ASO probes were tested of which a set of 35 SSO Probes are shown in Figure 6E as examples. In order to amplify a target gene or sequence to a detectable level for hybridization, multiplex PCR amplifications are carried out with a set of primers as shown in Figure 6F. Primerl-f, Primer2-f and Primer3-f are used for forward priming. Primers4-r, Primer5-r and Primer6-r are used for reversed priming. These primer pairs are able to generate for reversed priming. These primer pairs are able to generate about 264bp amplicons of 5' end-labeled for hybridization with color development to identify the genotypes in question.

HLA class A and B genotype identification can be done the same way as above when appropriate primers pairs and a number of probes all of which having the unique sequences corresponding to the HLA class A or B are used. Such sequences can be found either from the literature as reported in which their identities have been proven. Alternatively one can design these sequences using the scheme disclosed in this invention depicted in Figure 1.

### EXAMPLE 4

### Analysis of Viral or Bacterial Panels

The flow-through array format can be used to identify different organisms of different sequences or different genotypes. Figure 9 shows an example of identification of HPV genotypes. Different probes having identified sequences unique to corresponding viral types were immobilized on membrane for target capture. Samples to be analyzed were then amplified by PCR or other labeling and amplification methods. Addition of a generic probe would provide a mean to capture any other HPV types that are not covered for typing. This is very important and unique for HPV because it can capture many of the rare genotypes that cannot be identified by most HPV genotyping assay kits. HPV is highly heterogeneous, having more than 200 types of which only about 40 of them are considered clinically significant and the most common 20 cover up to 95% of cases in most populations. Hence the generic probe is important because it can capture these rare genotypes.

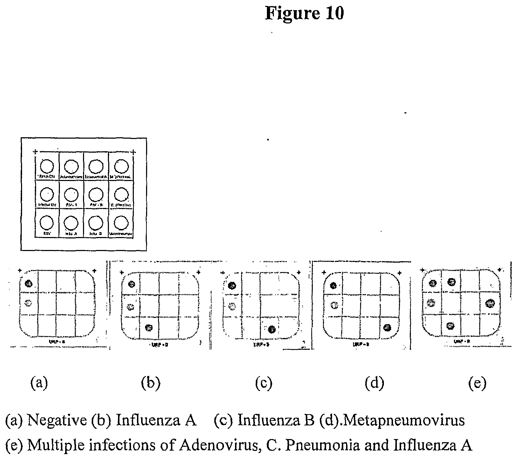

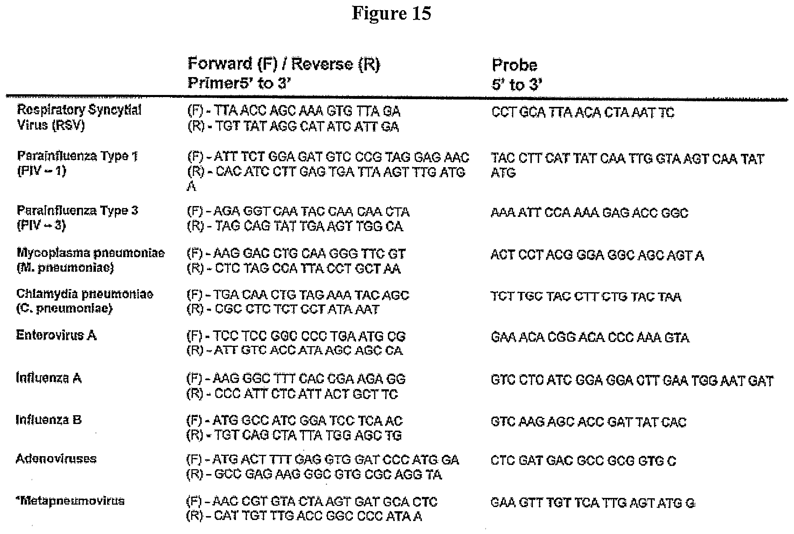

Figure 10 shows the identification of upper respiratory tract viral pathogens (Grondahl et. al., 1999; Mackay et. al., 2003). This illustrates a viral panel example in which many viruses having similar sequences or function belonging to the same class of pathogen can be identified using this illustration are given in Figure 15. However, other primers and capture probe sequences can be designed according to the scheme in Figure 1. This flow-through format has been used to successfully develop a fast test kit for SARS genotyping and was included into the URP array. However, since SARS is highly infectious and considered dangerous, the test kit is in a separate category.

Other panels identifying pathogens such as STD, hepatitis or bird flu such as H5N1 or related influenza viruses can also be developed for detection in similar manner.

### EXAMPLE 5

### Cancer Early Detection Panels

The cause of cancer is undoubtedly genetic in origin. Therefore if any genetic trait is identified, early detection is possible before tumorgenesis progresses into disease stages, which normally takes many years. The majority of cases in cancers are sporadic cases, due to either somatic mutations or structural modification of genes during one's life span. The longer one lives, the higher the concentration of mutated gene(s) and/or regulatory sequences will accumulate in an individual. Consequently, functionally defective phenotype (by either reducing the level of expression or producing defective gene product) shall occur leading to the onset of tumorgenesis. Thus in principle, genetic profile of a group of genes can be generated in the form of (i) expression level; (ii) the accumulation of defective products or (iii) the presence of genetic alterations such as (a) DNA mutation(s) in the responsible gene(s) and/or (b) alteration of the controlling region(s) - the promoter or enhancer. Such profile should provide insights into when and how tumorgenesis may have occurred and present diagnostic and prognostic assays.

Indeed mutation(s) of various genes have been identified to have definitive association with certain cancers such as the BRCAs gene for breast cancer; APC gene for colon cancer; p53 gene and Kras for cancers of multiple origins in human. Moreover, hypermethylation in the promoter region mostly located in the CpG islands will lead to silencing of tumor suppressor genes and induction of sporadic cancer. Since alteration(s) in the DNA is the prerequisite condition for cancers, detection of such mutation(s) and/or methylation status would be the earliest possible assay for preventive healthcare.

Recently, activities on epigenetic studies have gained a lot of momentum. Hypermethylation of a number of genes have been reported and that hypermethylation on these genes was found to be closely linked to cancer progression. These have led to the development of Methylation Specific Quantitative Real Time PCR (MSQ-PCR) in an attempt to give early diagnosis (Lo et.al., 1999; Facker et. al., 2006). However, despite its sensitivity, MSQ-PCR has its limitations. Firstly, the maximum number of color dyes is 6 and therefore only 3 genes' CpG islands can be tested simultaneously in a single reaction mixture. This is far less than the number of genes required to produce accurate and meaningful result. Secondly, since sample is often limited in quantity, it is not possible to repeat tests on other genes to confirm the presence or absence of cancer and to identify the type and site of cancer present.

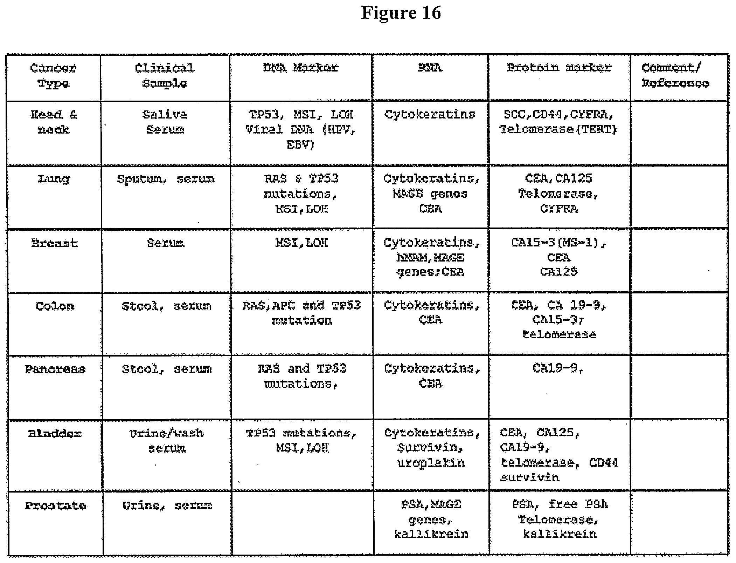

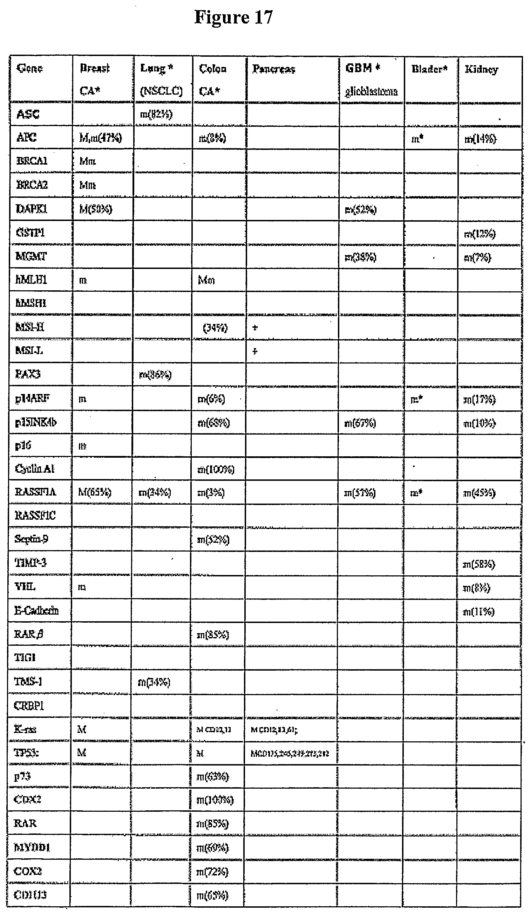

In contrast, examples shown here in this invention can be done by multiplex amplification and array assay which can screen a high number of genes or sequences simultaneously, giving a much higher probability for early cancer diagnosis. Figure 16 shows some of the cancer markers thought to be associated with cancers (Sidransky, 2002). Figure 17 provides a partial list of the genes responsible for regulation of metabolic pathways leading to cell homeostasis. Disruption in expression of these genes would lead to uncontrolled growth and cancers (Shinozaki et. al., 2005; Yu et. al., 2004). Using flow-through arrays mutation profiles as well as hypermethylation profiles, the present invention would accurately determine gene expression profiles for cancer diagnosis.

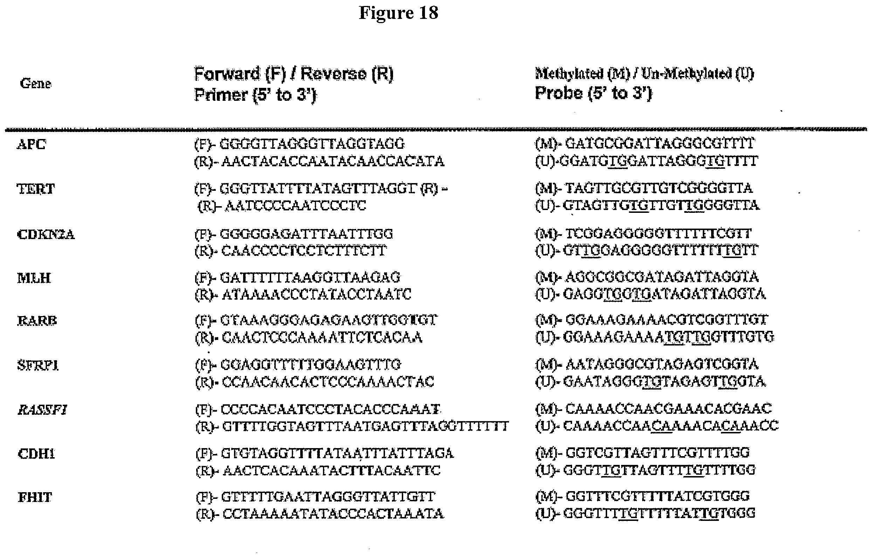

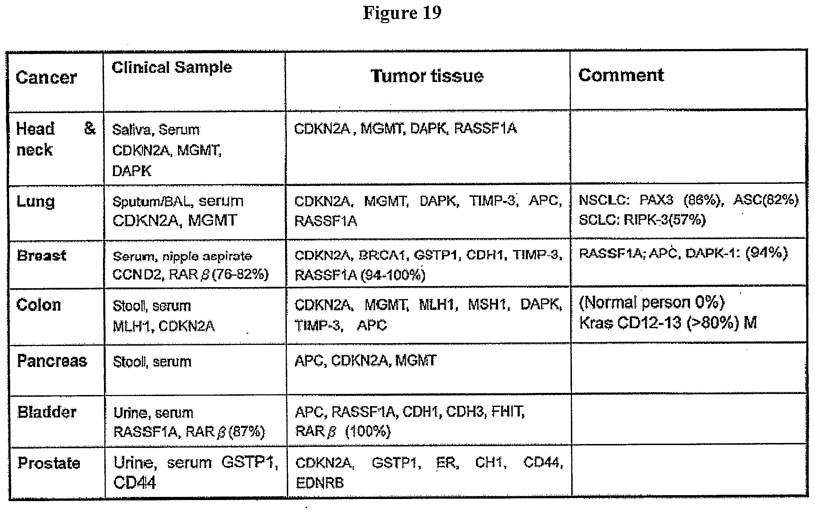

Figure 11 shows examples of different array formats for generating gene profiles on mutation(s) and on methylation(s). The assays provide DNA analyses and expression profiles by the detection of mRNAs. Cancers that occur in different parts of the human body (or organ) are expected to have different gene expression profiles. The methods and devices disclosed herein would provide distinctive profiles on gene mutations and/or the extend of methylation of CpG islands on a set of corresponding genes that will have significant diagnosis and prognosis value for the identification of cancer in different parts of the human body. Figure 18 shows some of the primer and probe sequences used for methylation detection. Some of the prospective markers and genes for methylation assays and their possible association to cancers are listed in Figure 19. Results from flow-through arrays experiments suggested that using such profiles provided more sensitive and specific assays for cancer diagnosis. It is specifically important to point out that DNA methylation can provide a definitive way to confirm any suggestive results from Biomarker assays. Examples of biomarker detection are given in Example 6 below.

### EXAMPLE 6

### New Embodiment of Device Designs

12 shows an alternative embodiment for Lateral flow-through assay disclosed herein. This new embodiment can be made as a new device that includes the essential elements, namely (i) the electronic control unit for operations; (ii) temperature block; (iii) the reaction chamber; and (iv) liquid delivery system. However, as depicted the reaction chamber where the membrane is located can be designed as a new separate unit, a disposable unit that can be fitted into the existing Flow-through Device by modifying its temperature block to accept such unit to achieve the required flow-through operation, manual or automation. The flow direction, the speed of flow and the sequence of solution reagents flow can be controlled accurately by the controller. This disposable reaction chamber can be made as single or multiple cassettes for separate or parallel reactions to provide increase in throughput. Evidently this embodiment can provide optimal conditions for effective detection with increase in sensitivity and specificity. The disposable as well as the totally enclosed setting in this embodiment can prevent any possibility of cross contamination. This system is well suited for clinical diagnoses that involve target amplification process and would reduce the risk of false positive results. Hence this new and novel embodiment disclosed herein is unique and is a very significant improvement for rapid flow-through assays.

### EXAMPLE 7

### One-Step Flow-Through Assay

Conventional hybridization and related assays are very time consuming because of complicated procedures that involve many separate steps. The Flow-through process and the device described herein have simplified the operation and resulted in substantial reduction of time and reagents cost without scarifying sensitivity and specificity for detection. The invention disclosed herein would provide further procedural improvements and expand the scope of testing.

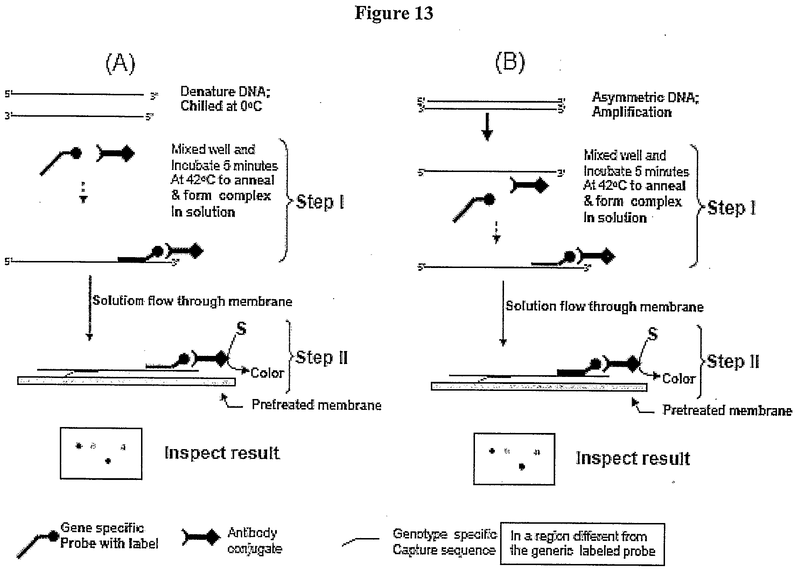

Figure 13 shows the scheme of a sample protocol: (i) after amplification, the amplicons are denatured, chilled to prevent self annealing, then mixed with hybridization reagents and incubated at predetermined hybridization temperature for 5 minutes before being dropped into the reaction site (the membrane) for capture and signal inspection; (ii) signal development by adding substrate for color development.

Other than those outlined in Figure 13, the following steps will help to achieve better results: (a) asymmetric amplification by PCR or equivalent method to generate more single strand copies in complementary to the capture probe(s); (b) strepavidin labeling for signal generating tags and this conjugate shall be used for interacting with the biotin-labeled target DNA molecule to give signal; and (c) the number of signal development procedures will depend on the kind of labeling tags either during amplicon generation or the signal developing conjugate. The use of fluorescence dyes in amplicon production or direct color labeling of the conjugate will eliminate subsequent color development steps after hybridization. Otherwise similar color development steps by conventional enzyme-linked conjugate plus substrate can be used.

Besides fluorescence tags, a single-step hybridization process can label target sequences or molecules with quantum dot, colloidal gold particles, magnetic particles or other appropriate labeling tags to eliminate the enzyme-linked conjugate substrate color development step. These improvements will enable a technician to complete the entire hybridization process and signal production in 5 minutes or less. Hence, the method of the present invention should provide further savings in terms of time and reagent cost.

Figure 13 illustrates an example of how the single-step hybridization can be performed. With adequate concentration of analytes (either in the form of sample volume or concentration, i.e. if the total number of analytes molecules is enough) and appropriate signal generating labeling tags, direct analyses on the original sample without amplification may be possible by flowing the sample solution (after thoroughly mixed well with all reagents to provide analyzable complexes) continuously through the membrane on which target molecules have been captured by immobilized probes to generate detectable signal similar to the over-the-counter immunochemical strip testing kits.

### EXAMPLE 8

### A Novel Signal Amplification Assay

Contamination can be a serious problem for PCR reactions. Hence, instead of product amplification by PCR, molecular biology or DNA-based diagnosis can be enhanced by an alternative strategy of signal amplification. Branch DNA (b-DNA) has been and still is being used with certain success. DNA super molecular complexes are also under investigation. Recently the hybrid capture technology for HPV detection is another example. Unfortunately, these methods are neither suitable nor applied to membrane-based assays.

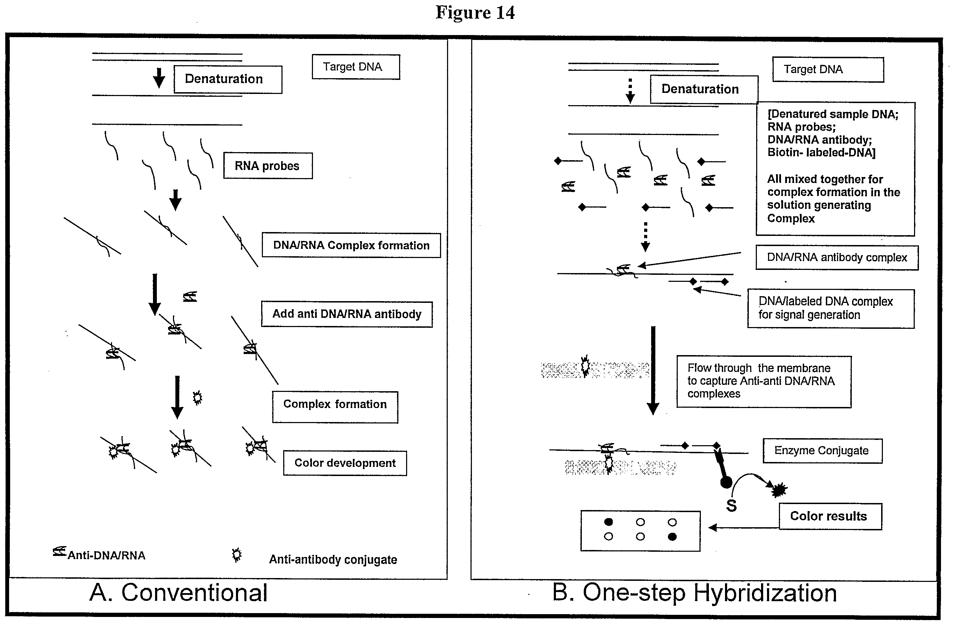

The present invention presents examples of membrane-based application using the flow-through platform. As shown in Figure 14, the detection system includes: (1) sequence-specific capturing oligo-probes immobilized onto membrane as detection arrays; (2) specific RNA or DNA oligos are designed for binding with target DNA molecules in the sample solution; (3) polyclonal antibody specific to DNA/RNA molecules, having high affinity to DNA/RNA complex in general but not sequence specific for capturing the DNA/RNA molecule in target sample solution; (4) antibody specific to anti-DNA/RNA for binding and concentrating the antibody-DNA/RNA complex in the target solution; (5) signal generating tags-labeled DNA molecules in the region(s) other than those used for the captured RNA and DNA oligos immobilized on the membrane; and (6) reagents for signal development and device.

As shown in Figure 14, the method steps include: (1) DNA isolated and purified from target sample in adequate quantity is denatured, chilled and mixed with RNA capture oligos hybridized in the presence of appropriate amount of anti-DNA/RNA antibody to form complex; (2) the complex is then recovered by affinity column to be concentrated;(3) the complex is re-suspended, equilibrated at required temperature and flow through the membrane for hybridization and wash; (4) DNA tags is added, washed, followed by signal development and report. The procedure described above is the initial experimental approach. Further optimization can be done in accordance to the concept and spirit of this invention.

In principle, single step is possible by putting in appropriate components together in the sample solution (i.e. after deproteinization to exclude cell debris and non-nucleic acid complexes) and flow into the membrane for hybridization capture. The present invention employs detection assay direct from the neat solution sample (the starting sample) without amplification. It is applicable and enabled using the flow-through system because there is no limitation on the solution volume used. The sensitivity of detection varies according to the signal generating system used. For example, using the AP-AV and color development system we had achieved 0.3 fetomole/label. At least ten-fold increase in sensitivity can be achieved by chemiluminescence assay. By increasing the number of label molecule in the signal DNA tag, detection in the range of attomole is achievable in principle. At this concentration many of the viral infections can be readily detected.

### EQUIVALENTS

Those skilled in the art will recognize, or be able to ascertain using no more than routine experimentation, many equivalents to the specific embodiments of the invention described herein. Such equivalents are intended to be encompassed by the following claims.

### References

Awift-Scanlan et. Al., two-color quantitative multiplex methylation-specific PCR, BioTechniques 2006, 40: 210-219.
Brightwell et al. SNP genotyping using a simple and rapid single-tube modification of ARMS illustrated by analysis of 6 SNPs in a population of males with FRAXA repeat expansions. Mol Cell Probes. 2002 Aug;16(4):297-305.
Bunce M et al. (1995) Tissue Antigens, 46, 355-367.
Chakraborty R, Stivers D N. Paternity exclusion by DNA markers: effects of paternal mutations. J Forensic Sci 1996 July; 41(4): 671-7.
Chow R and Tonai R., "High throughput methods for HLA typing", US Pat. No. 6,670,124, December 30, 2003.
Edwards A, Civitello A, Hammond H A, Caskey C T. DNA typing and genetic mapping with trimeric and tetrameric tandem repeats. Am J Hum Genet. 1991 October; 49(4): 746-56.
Facker MJ et. al., Quantitative Multiplex methylation-specific PCR Analysis Doubles detection of Tumor cells in breast Ductal fluid. Clinical Cancer Res. 2006; 12 June 1, 3306-3310.
Gill P, Jeffreys A J, Werrett D J. Forensic application of DNA 'fingerprints'. Nature. 1985 December 12-18; 318(6046): 577-9.
Grondahl et. al., Journal of Clinical Microbiology 1999 Jan;37: 1-7
Kaneshige T et. al., Rapid and Practical HLA Class II Genotyping by Reversed Dot Blotting, Transplantation Proceedings, 1993 Feb.; 25(1): 194-198.
Lo et.al., Quantitative analysis of aberrant p16 methylation using real-time quantitative methylation-specific polymerase chain reaction, Cancer Res. 1999 Aug 15, 59: 3899-903.
Mach B et al. (1990) in Molecular Biology of HLA Class II Antigens, ed. Silver J (CRC, Boca Raton, FL), pp. 201-223.
Mach et al., "DNA sequences coding for the DR beta-chain locus of the human lymphocyte antigen complex and polypeptides, diagnostic typing processes and products related thereto", US Pat. No. 6,818,393, November 16, 2004.
Mackay et. al. Journal of Clinical Microbiology 2003 Jan; 100-105
Robinson J, Matthew J. Waller, et al.,IMGT/HLA and IMGT/MHC: sequence databases for the study of the major histocompatibility complex, Nucleic Acids Res. 2003 Jan 1;31(1):311-4.
Robinson J, Waller MJ, Parham P, Bodmer JG, Marsh SG., IMGT/HLA Database--a sequence database for the human major histocompatibility complex, Nucleic Acids Res. 2001 Jan 1;29(1):210-3.
Shinozaki M et. al., Distinct Hypermethylation Profile of primary Breast Cancer Is Associated with Sentinel Lymph Node Metastasis, Clin. Cancer Res. 2005 March 15, 11; 2156-2162.
Sidransky D., Emerging Molecular Markers of cancer , 2002 March, 2; 210-219.
Tam, Flow Through Nucleic Acid Hybridization Device, U.S. Pat. No. 5,741,647.
Tam, Flow Through Nucleic Acid Hybridization Device, U.S. Pat. No. 6,020,187.
Tam, DNA Fingerprinting Using Allelic Specific Oligonucleotide Reversed Dot Blot (ASO RDB) Flow Through Hybridization Process And Device, U.S. Publication No. 2005/0079493, April 14, 2005.
Tam, SNP-BASED HLA-DP, DR And DQ Genotyping Analysis By Reverse Dot Blot Flow Through Hybridization, U.S. Publication No. 2004/0209253, October 21, 2004.
Tam, Rapid Genotyping Analysis and the Device Thereof, U.S. Publication No. 2006/0292601, December 28, 2006.
Thomas ED (1983) J. Clinical Oncology 1, 517-531.
Weiss K M. In search of human variation. Genome Res 1998 July; 8(7): 691-7
Yu J et. Al., Methylation profiles of the four promoter-CpG islands and concordant methylation behaviour of sixteen genes that may contribute to carcinogenesis of astrocytoma, BMC Cancer 2004, 4: 65-79.
Zhao L P, Aragaki C, Hsu L, Quiaoit F. Mapping of complex traits by single-nucleotide polymorphisms. Am J Hum Genet 1998 July; 63(1): 225-40.

## Claims

1. A method of performing rapid nucleic acid detection, comprising the steps of:
(a) obtaining a sample comprising a target nucleic acid molecule;
(b) mixing the target nucleic acid molecule with (i) a first probe that will bind to the nucleic acid molecule, and (ii) a second agent that will bind to the first probe, thereby forming a nucleic acid molecule complex in solution, wherein the nucleic acid molecule complex comprises the first probe and the second agent, the second agent comprising a signal generating labeling tag or an enzyme-linked conjugate;
(c) applying in a flow through manner the solution comprising the nucleic acid molecule complex to an array comprising a third probe that will bind to the complex, thereby capturing the nucleic acid complex on the array; and
(d) detecting the captured nucleic acid molecule on the array.

2. The method of claim 1, wherein the labelling tag includes colloidal gold, fluorescent tag, quantum dot or magnetic particle.

3. The method of claim 1, wherein the first probe and the third probe bind to different regions of the target nucleic acid molecule.

4. The method of claim 1, wherein the method further comprises a step of asymmetric amplification to generate single strand copies of the target nucleic acid molecule.

5. The method of any one of claims 1 to 4, wherein:
- the first probe will form a complex with the target nucleic acid molecule;
- the second agent is an enzyme-linked conjugate comprising a first antibody and a labelling agent, wherein the first antibody will bind to said complex; and
- the third probe is a second antibody that will bind to the first antibody, thereby capturing the nucleic acid molecule complex on the array.

6. A method according to claim 5, wherein the enzyme is for color development.

7. A lateral flow hybridization device for detecting target analytes of nucleic acid molecules in a solution, the device comprising:
(a) a membrane for immobilizing capture molecules capable of capturing target analytes, wherein the membrane is positioned in a reaction chamber where it can be maintained in controlled temperature conditions;
(b) controlling elements that can be regulated to maintain the reaction chamber in the controlled temperature conditions;
(c) connecting elements for connection to a power supply and control unit that can regulate and maintain the controlled temperature conditions; and
(d) liquid delivery elements capable of accepting and removing solution to and from the reaction chamber, wherein the device is adapted such that, in use, the solution is maintained in a lateral flow direction that allows the solution flows through the membrane pores in cross-section so that all target analytes pass through the capture molecules immobilized on the membrane, thereby providing higher sensitivity of analytes detection;
**characterized in that** the reaction chamber comprises a heat conduction plate which is in contact with heating and cooling elements, wherein the heat conduction plate is adapted to be in contact with detection membrane strips on the membrane during use when the target analytes flow laterally through the membrane.

8. The device of claim 7, wherein the device comprises a temperature block for receiving the reaction chamber, and the temperature block is adapted for heating the membrane and connected to a power supply and control unit for controlling heating of the reaction chamber.

9. The device of claims 7 or 8, wherein the membrane comprises an array or arrays of capture molecules for capturing and detecting target analytes and is arranged such that, in use, the solution flows from one end to another end of the array, and wherein the capture molecules are arranged into a linear dot-matrix array.

10. The device of claim 7, wherein the membrane is formed of a material containing nitrocellulose, nylon, Nytron, Biodyne,' or Porex.

11. The device of any of claims 7 to 10, wherein accepting and removing solution to and from the reaction chamber is by regulated liquid pumping.

12. The device of claim 11, wherein the liquid pumping is used to recirculate solution containing the target analytes through the membrane.

13. The device of any one of claims 7 to 12 including more than one of said reaction chambers, wherein the reaction chambers are arranged as disposable cassettes.

14. A lateral flow hybridization system comprising a plurality of hybridization devices of any one of claims 7 to 13, wherein the hybridization devices are connected to a power supply and control unit capable of supplying energy and providing regulatory control to the hybridization devices.

15. The system of claim 14, wherein each of the hybridization devices is controlled independently by the power supply and control unit so that each hybridization device can perform different analysis under different conditions.

## Patentansprüche

1. Verfahren zur Durchführung eines schnellen Nukleinsäure-Nachweises, folgende Schritte umfassend:
a) Erhalt einer Probe, die ein Zielnukleinsäuremolekül umfasst;
b) Mischung des Zielnukleinsäuremoleküls mit (i) einer ersten Sonde, die sich an das Nukleinsäuremolekül bindet, und (ii) einem zweiten Wirkstoff, der sich an die erste Sonde bindet, wodurch ein Nukleinsäuremolekül-Komplex in Lösung gebildet wird, wobei der Nukleinsäuremolekül-Komplex die erste Sonde und den zweiten Wirkstoff umfasst und wobei der zweite Wirkstoff ein signalerzeugendes Markierungs-Tag oder ein enzymgebundenes Konjugat umfasst;
c) Anwendung der den Nukleinsäuremolekül-Komplex umfassenden Lösung in Form eines Durchflusses auf einen Array umfassend eine dritte Sonde, die sich an den Komplex bindet, wodurch der Nukleinsäurekomplex auf dem Array eingefangen wird; und
d) Nachweis des eingefangenen Nukleinsäuremoleküls auf dem Array.

2. Verfahren nach Anspruch 1, wobei das Markierungs-Tag kolloidales Gold, eine Fluoreszenzmarkierung, einen Quantenpunkt oder ein Magnetpartikel aufweist.

3. Verfahren nach Anspruch 1, wobei die erste Sonde und die dritte Sonde sind an unterschiedliche Regionen des Zielnukleinsäuremoleküls binden.

4. Verfahren nach Anspruch 1, wobei das Verfahren weiterhin einen Schritt der asymmetrischen Amplifikation zur Erzeugung von Einzelstrangkopien des Zielnukleinsäuremoleküls umfasst.

5. Verfahren nach einem der Ansprüche 1 bis 4, wobei:
- die erste Sonde einen Komplex mit dem Zielnukleinsäuremolekül bildet;
- der zweite Wirkstoff ein enzymgebundenes Konjugat umfassend einen ersten Antikörper und einen Markierungswirkstoff ist, wobei der erste Antikörper sich an diesen Komplex bindet; und
- die dritte Sonde ein zweiter Antikörper ist, der sich an den ersten Antikörper bindet, wodurch der Nukleinsäuremolekül-Komplex auf dem Array eingefangen wird.

6. Verfahren nach Anspruch 5, wobei das Enzym zur Farbentwicklung vorgesehen ist.

7. Lateral-Flow-Hybridisierungsvorrichtung zum Nachweis von Zielanalyten von Nukleinsäuremolekülen in einer Lösung, wobei die Vorrichtung Folgendes umfasst:
a) eine Membran zur Immobilisierung von Fängermolekülen, die zum Einfang von Zielanalyten fähig sind, wobei die Membran in einer Reaktionskammer positioniert ist, in der sie unter kontrollierten Temperaturbedingungen gehalten werden kann;
b) Steuerelemente, die geregelt werden können, um die Reaktionskammer in den kontrollierten Temperaturbedingungen zu halten;
c) Anschlusselemente zum Anschluss an eine Stromversorgung und eine Steuereinheit, welche die kontrollierten Temperaturbedingungen regeln und aufrechterhalten können; und
d) Flüssigkeitsabgabeelemente, die zur Annahme und Entfernung von Lösung zu und von der Reaktionskammer fähig sind, wobei die Vorrichtung so ausgelegt ist, dass die Lösung bei Verwendung in einer lateralen Flussrichtung gehalten wird, welche die Lösungsflüsse durch die Membranporen im Querschnitt erlaubt, sodass alle Zielanalyten durch die immobilisierten Fängermoleküle auf der Membran passieren, wodurch eine höhere Empfindlichkeit des Analytenachweises bereitgestellt wird,
**dadurch gekennzeichnet, dass** die Reaktionskammer ein Wärmeleitblech umfasst, das in Kontakt mit Heiz- und Kühlelementen ist, wobei das Wärmeleitblech dazu ausgelegt, ist, während der Verwendung in Kontakt mit Nachweismembranstreifen auf der Membran zu sein, wenn die Zielanalyten lateral durch die Membran fließen.

8. Vorrichtung nach Anspruch 7, wobei die Vorrichtung einen Temperaturblock zur Aufnahme der Reaktionskammer umfasst und der Temperaturblock zur Erhitzung der Membran ausgelegt und an eine Stromversorgung und einer Steuereinheit zur Steuerung der Erwärmung der Reaktionskammer angeschlossen ist.

9. Vorrichtung nach Anspruch 7 oder 8, wobei die Membran einen Array oder Arrays von Fängermolekülen zum Einfangen und Nachweis von Zielanalyten umfasst und so angeordnet ist, dass die Lösung bei Verwendung von einem Ende zu einem anderen Ende des Arrays fließt, und wobei die Fängermoleküle in einem linearen Punktmatrix-Array angeordnet sind.

10. Vorrichtung nach Anspruch 7, wobei die Membran aus einem Material gebildet ist, das Nitrocellulose, Nylon, Nytron, Biodyn oder Porex enthält.

11. Vorrichtung nach einem der Ansprüche 7 bis 10, wobei die Annahme und Entfernung von Lösung zu und von der Reaktionskammer durch geregeltes Flüssigkeitspumpen erfolgt.

12. Vorrichtung nach Anspruch 11, wobei das Flüssigkeitspumpen zur Rezirkulation der die Zielanalyten enthaltenden Lösung durch die Membran verwendet wird.

13. Vorrichtung nach einem der Ansprüche 7 bis 12, die mehr als eine dieser Reaktionskammern aufweist, wobei die Reaktionskammern als Einwegkassetten angeordnet sind.

14. Lateral-Flow-Hybridisierungssystem, umfassend eine Vielzahl von Hybridisierungsvorrichtungen nach einem der Ansprüche 7 bis 13, wobei die Hybridisierungsvorrichtungen an eine Stromversorgung und Steuereinheit angeschlossen sind, die zur Energieversorgung und Bereitstellung einer regelnden Steuerung der Hybridisierungsvorrichtungen fähig sind.

15. System nach Anspruch 14, wobei jede der Hybridisierungsvorrichtungen unabhängig von der Stromversorgung und Steuereinheit gesteuert wird, sodass jede Hybridisierungsvorrichtung unterschiedliche Analysen unter unterschiedlichen Bedingungen durchführen kann.

## Revendications

1. Procédé d'exécution de la détection des acides nucléiques, comprenant les étapes consistant à:
(a) obtenir un échantillon comprenant une molécule d'acide nucléique cible ;
(b) mélanger la molécule d'acide nucléique cible avec (i) une première sonde qui va se lier à la molécule d'acide nucléique et (ii) un second agent qui va se lier à la première sonde, ce qui permet de former un complexe de molécules d'acide nucléique en solution. Le complexe de molécules d'acide nucléique comprend la première sonde et le second agent, ce dernier comprenant un marqueur générateur de signaux ou un conjugué enzymatique ;
(c) appliquer par écoulement la solution comprenant le complexe de molécules d'acide nucléique à un ensemble comprenant une troisième sonde qui va se lier au complexe, ce qui permet de capturer le complexe d'acides nucléiques sur l'ensemble ; et
(d) détecter la molécule d'acide nucléique capturée sur l'ensemble.

2. Procédé selon la revendication 1 dans lequel le marqueur inclut de l'or colloïdal, un marqueur fluorescent, une boîte quantique ou une particule magnétique.

3. Procédé selon la revendication 1 dans lequel la première sonde et la troisième sonde se lient à différentes régions de la molécule d'acide nucléique cible.

4. Procédé selon la revendication 1 dans lequel le procédé comprend en outre une étape d'amplification asymétrique pour générer des copies à simple brin de la molécule d'acide nucléique cible.

5. Procédé selon l'une quelconque des revendications 1 à 4 dans lequel :
- la première sonde va former un complexe avec la molécule d'acide nucléique cible ;
- le second agent est un conjugué enzymatique comprenant un premier anticorps et un agent de marquage, où le premier anticorps va se lier audit complexe ; et
- la troisième sonde est un second anticorps qui va se lier au premier anticorps, ce qui permet de capturer le complexe de molécules d'acide nucléique sur l'ensemble.

6. Procédé selon la revendication 5 dans lequel l'enzyme est destiné au développement de la couleur.

7. Dispositif d'hybridation à flux latéral pour la détection des analytes cibles des molécules d'acide nucléique en solution, le dispositif comprenant :
(a) une membrane pour l'immobilisation des molécules de liaison capable de capturer des analytes cibles, la membrane étant positionnée dans une chambre de réaction où elle peut être maintenue dans des conditions de température contrôlée ;
(b) des éléments de contrôle qui peuvent être régulés pour maintenir la chambre de réaction dans les conditions de température contrôlée ;
(c) des éléments de connexion pour le raccordement à une unité d'alimentation et de commande qui peuvent réguler et maintenir les conditions de température contrôlée ; et
(d) des éléments de distribution liquide capables d'accepter et de retirer la solution dans et de la chambre de réaction, le dispositif étant adapté de telle façon que, lorsqu'il est utilisé, la solution est maintenue dans un sens de flux latéral qui permet à la solution de s'écouler à travers les pores de la membrane en section transversale de sorte que tous les analytes cibles passent à travers les molécules de liaison immobilisée sur la membrane, ce qui permet d'assurer une plus grande sensibilité de détection des analytes ;
**caractérisé en ce que** la chambre de réaction comprend une plaque de conduction thermique qui est en contact avec des éléments de chauffage et de refroidissement. La plaque de conduction thermique est adaptée pour être en contact avec les bandes de membrane de détection sur la membrane au cours de l'utilisation, lorsque les analytes cibles s'écoulent latéralement à travers la membrane.

8. Le dispositif selon la revendication 7 dans lequel le dispositif comprend un bloc de température pour accepter la chambre de réaction. Le bloc de température est adapté pour le chauffage de la membrane et est raccordé à l'unité d'alimentation et de commande pour le contrôle du chauffage de la chambre de réaction.

9. Le dispositif selon la revendication 7 ou 8 dans lequel la membrane comprend un ensemble ou des ensembles de molécules de liaison pour la capture et la détection des analytes cibles et est agencé de sorte que, lors de l'utilisation, la solution s'écoule d'une extrémité à l'autre de l'ensemble, les molécules de liaison étant disposées dans un ensemble matriciel linéaire.

10. Le dispositif selon la revendication 7 dans lequel la membrane est constituée d'un matériau contenant de la nitrocellulose, du nylon, du Nytron, de la Biodyne ou du Porex.

11. Le dispositif selon l'une quelconque des revendications 7 à 10 dans lequel l'acceptation et le retrait de la solution dans ou de la chambre de réaction se font par pompage liquide régulé.

12. Le dispositif selon la revendication 11 dans lequel le pompage liquide est utilisé pour la recirculation de la solution contenant les analytes cibles à travers la membrane.

13. Le dispositif selon l'une quelconque des revendications 7 à 12 incluant plus desdites chambres de réaction, où les chambres de réaction sont disposées en tant que cassettes jetables.

14. Un système d'hybridation à flux latéral comprenant une pluralité de dispositifs d'hybridation selon l'une quelconque des revendications 7 à 13, où les dispositifs d'hybridation sont raccordés à une unité d'alimentation et de commande capable de fournir l'énergie et d'assurer la commande régulatrice des dispositifs d'hybridation.

15. Le système selon la revendication 14 dans lequel chacun des dispositifs d'hybridation est commandé indépendamment par l'unité d'alimentation et de commande de sorte que chaque dispositif d'hybridation puisse exécuter différentes analyses dans des conditions différentes.
